# EUROPEAN PATENT APPLICATION

(11) **EP 1 445 320 A1**
(43) Date of publication of application: **11.08.2004**
(21) Application number: 03002424.4
(22) Date of filing: 05.02.2003
(51) Int. Cl.: C12N 15/65, C12N 15/82, C12N 15/90

(54) **Automated gene-targeting using non-toxic detectable markers**

(71) Applicant: ARTEMIS Pharmaceuticals GmbH, 51062 Köln (DE)
(72) Inventor: Kauselmann, Gunther, 50676 Köln (DE); Zevnik, Branko, 51375 Leverkusen (DE); Seibler, Jost, 50733 Köln (DE); Kern, Heidrun, 40227 Düsseldorf (DE)
(74) Representative: Helbing, Jörg, Dr. Dipl.-Chem.

(57) **Abstract**

The invention relates to a method that enables automated identification and isolation of cells harbouring a predetermined genetic modification (homologous recombination) using detectable/sortable markers, e.g. fluorescence markers, to identify homologous DNA modifications. Suitable vectors are also provided.

## Description

The invention relates to a method that enables automated identification and isolation of cells harbouring a predetermined genetic modification (homologous recombination) using detectable/sortable markers, e.g. fluorescence markers, to identify homologous DNA modifications. Suitable vectors are also provided.

### Background

Gene targeting in cells, including embryonic stem (ES) cells, relies on the homologous recombination (HR) between a native chromosomal gene and introduced exogenous DNA (Smithies, O. et al., Nature, 317(6034):230-4 (1985); Thomas, K.R. et al., Cell, 44(3):419-28 (1986); Doetschman, T. et al., Proc. Natl. Acad. Sci. USA 85(22):8583-7 (1988); Thomas, K.R., Capecchi, M.R., Cell, 51(3):503-12 (1987)). The method requires transfection of the foreign DNA (targeting vector) into the ES cells, usually by electroporation. HR is a very inefficient process, working in the range of 1 x 10⁻⁵ - 1 x 10⁻⁶ events per electroporated ES cells (for review see Hooper, M.C., Harwood, Embryonal Stem Cell: Introducing Planned Changes into the Animal Geneline, New York (1993)). Most genes of interest are not directly selectable. The identification of homologous recombined (gene targeted) ES cells therefore require the application of selection mechanism.

Positive selection is usually achieved by incorporation of an antibiotic resistance gene into the targeting vector, located between at least 2 stretches of DNA homologous to the targeting region. Upon addition of antibiotic to the culture medium of electroporated ES cells, expression of the positive selection marker allows survival only of those cells which have stably introduced the electroporated DNA into their genome.

Additionally, negative selection strategies further enrich selective growth and identification of HR events. A negative selection marker is added at the 5' or 3' end of a homologous DNA stretch (Mansour, S.L. et al., Nature, 336:348-352 (1988); U.S. Patents Nos. 5,487,992 and 5,464,764). Non-homologous DNA regions outside the homologous regions are not incorporated into the genome if HR has occurred. Current protocols (U.S. Patents 5,487,992 and 5,464,765) require the addition of a compound to the culture medium, to mediate selection against incorporation of the marker, effectively killing ES cells, which have undergone random recombination. Negative selection generally achieves enrichments of only 2-5fold (Sedivy, J. M., Dutriaux, A., Trends Genet., 15(3):88-90 (1999)).
Given optimal vector design to allow homologous recombination, the described selection strategies known in the art (e.g. the positive-negative selection strategies disclosed in U.S. Patents 5,487,992 and 5,464,765) allow identification of HR in about 5% of all analysed ES cells. Said selection strategies are certainly not optimal and it is therefore desirable to
(a) optimize enrichment of homologous recombinants,
(b) reduce variability in efficiency of the currently available methods for enrichment,
(c) avoid unwanted potential toxicity of agents for negative or negative and positive selection agents. Gancyclovir, the most widely used agent for negative selection, inhibits DNA polymerase and therefore cellular proliferation (Matthews, T., Boehme, R., Reviews of Infectious Diseases, 10(3):490-4 (1998)). Such inhibition may not be tolerable under GMP standards, in particular in potential applications of gene targeting for gene therapy in humans.

Moreover, the selection procedures known in the art requires ES cell culture over a period of 7 - 8 days, to allow selection to work, and clonal expansion of cells to an absolute number of ca. 2000, to enable molecular analysis and further expansion of the cell population. However, culture of ES cells requires adherent growth of the cells on defined substrate, usually mitotically inactivated "feeder cells". This, in turn, necessitates laborious, manual "picking" of identified ES clones from their substrate to enable individual clonal growth in culture vessels for maintenance and molecular analysis. It was therefore also desirable to reduce the time period required to isolate potential HR ES clones as well as the manual labour, i.e. picking of ES cell clones, i.e. by increasing the automation of the gene targeting process employing current protocols.

Fluorescent probes on the other side are known as a powerful tool for identification of molecular events in single cells. The most widely known is the green fluorescent protein (GFP) from bioluminescent jellyfish *Aequorea victoria*. However, approximately 30 distinct fluorescent proteins have been discovered and cloned from a variety of species (Labas, Y.A. et al., PNAS, 99(7):4256-4261 (2002)), (Matz, M.V. et al., Nat. Biotechnol., 17(10):906-18 (1999)) for review see: (Zhang, J. et al., Nat. Rev. Mol. Cell. Biol., 3(12):906-18 (2002 )).
For certain reasons such as the sensitivity of the cells to be transfected, optically detectable markers including fluorescent probes such as GFP and its variants were only seldom employed as detectable markers in gene targeting experiments. E. g. WO 02/06630 discloses that GFP might be utilized instead of a neomycin phosphotransferase gene in a gene targeting process similar to the basic positive selection protocol.

### Summary of the Invention

It was now found that certain vectors for targeted homologous recombination which contain one or more expression cassettes coding for a detectable marker and being placed outside the targeting cassette (i.e. the region of homology of the vector to the genomic DNA) allow rapid and reliable distinction, preferably visual distinction, between targeted and non-targeted ES cells. One example of such an optically detectable marker is ZsGreen (Clontech, Palo Alto, CA). Another example is the application of a first fluorescence marker gene (ZsGreen) outside the region of homology (i.e. in the expression cassette) and a second fluorescence marker gene differing from the first marker gene inside the region of homology (i.e. inside the targeting cassette). It was found that with such a gene targeting vector containing two different fluorescent markers identification of both (stably transfected) non-targeted and correctly targeted ES cells via optical detection was possible. Furthermore, employing Fluorescence Activated Cell Sorting (FACS) for identification allows plating of a population of ES cells enriched for gene-targeting events, in an individualized way. Altogether, this method allows for the first time automated gene-targeting starting with transfection and resulting in molecular analysis and/or cryoconservation of identified ES clones.

The invention thus provides
(1) a vector for targeted homologous recombination of eukaryotic cells comprising
   (A) a homologous targeting cassette harbouring a functional DNA segment and
   (B) an expression cassette harbouring a DNA sequence coding for a first detectable marker, said expression cassette (B) being connected with said homologous targeting cassette (A) so as to allow distinction between targeted and non-targeted cells;
(2) a method for homologous recombination of eukaryotic cells comprising
   (a) transfecting cells with a homologous targeting vector as defined in (1) above allowing distinction between targeted and non-targeted cells;
   (b) identifying and/or isolating cells containing the positive selection marker or the second detectable marker; and
   (c) identifying and isolating cells as homologous recombinants by the absence of the first detectable marker;
(3) a method for preparing transgenic tissues, organs and/or multi-cell organisms which comprises utilizing the method as defined in (2) above.

The vector of (1) above, if utilized in a homologous recombination process allows direct or indirect distinction between correctly targeted and non-targeted cells. Indirect distinction can be achieved via addition of non-toxic compounds to the cells or via metabolic or enzymatic action of the gene product (detectable marker) encoded by the expression cassette.

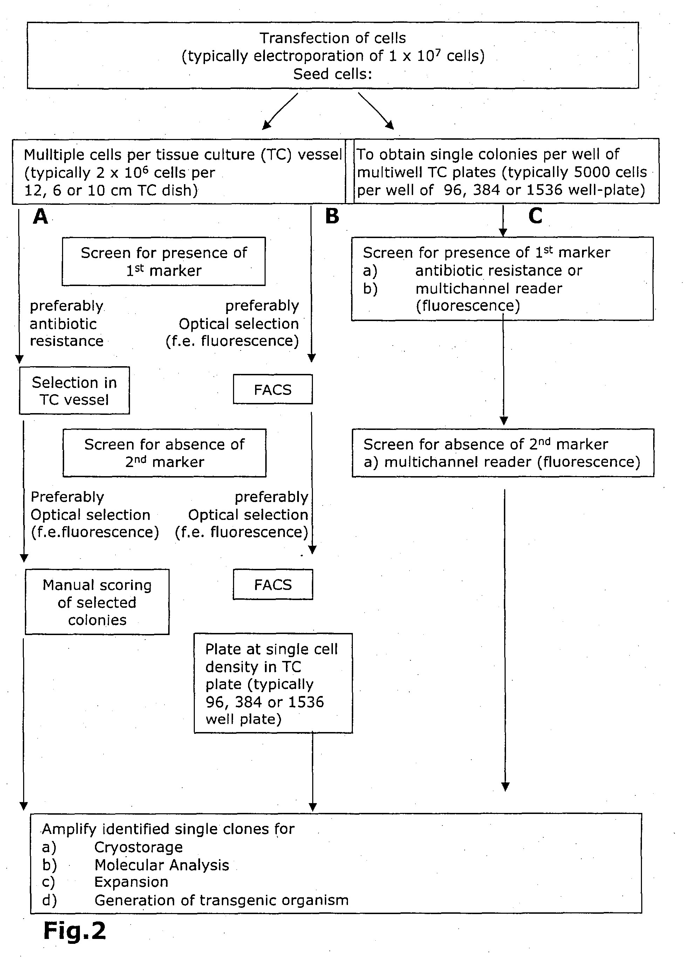

By utilizing one or more fluorescence markers as detectable markers, the method (2) of the invention
a) allows to optically distinguish random DNA integrants (light emission) and HR eukaryotic cells, including ES cells (no light emission) (Fig. 2);
b) enhances isolation and reduces variability of recovery of HR ES cells in individual experiments since the addition of compounds to the culture medium is avoided;
c) allows selective, automated distinction of growing transfected ES cell colonies by plating of ES cells, transformed with such light emission targeting vectors, in a way such that preferentially single cell clones grow within a spatially separated region (1 well of a multiwell-dish) and subsequent fluorescence analysis (such ES clones can then automatically be expanded and taken to molecular analysis (Fig. 2));
d) allows selective, automated sorting of growing transfected ES cells for the absence of the first detectable marker and/or the presence of the second detectable marker and/or by FACS analysis.

The method of the invention has the following advantages over current technology:
(i) The detection strategy allows selective identification of gene targeted ES clones without exposure of ES cells to potentially toxic chemicals, proteins or mutagens.
(ii) Automated expansion, isolation and molecular analysis of gene targeted ES clones upon electroporation.

### Short Description of the Figures

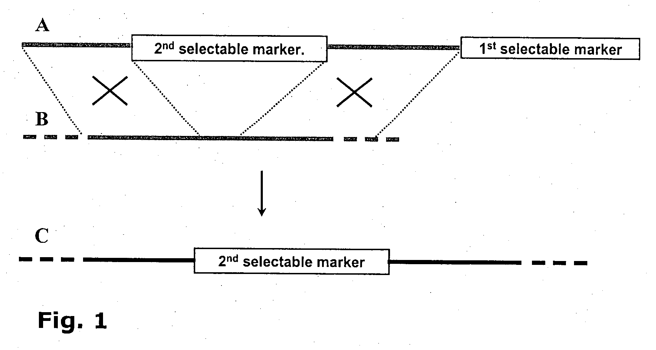

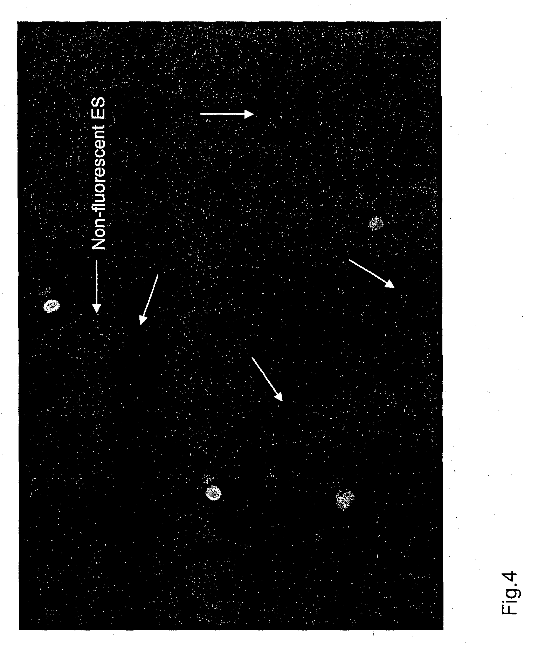

Fig 1: Selection mechanism in homologous recombination: A: targeting vector; B: homologous genomic DNA; C: homologous recombined DNA, presence of 2nd marker, absence of 1st marker.
Fig. 2: Scheme of manual and automated gene targeting process employing novel detectable markers.
Fig. 3: Generation of targeted ES cells. Scheme of the gene targeting strategy. Both constructs, (i) the targeting vector for positive and negative selection (pOMP1) (3A), and (ii) the targeting vector for positive and optical detection (pOMP3) (3B) were used for insertion of the neo gene into the *Rosa26* locus by homologous recombination as depicted. E: EcoRV; X: XbaI.
   (3C) Southern blot analysis of genomic DNA from ES cells transfected with constructs described in (Fig 4A) and (Fig 4B). The DNA of ES cells was digested with EcoRV and hybridized with probe 1 specific for the *rosa26* locus. The sizes of the wt and the targeted allele are 11.5 kb and 2.5 kb, respectively. The picture shows the analysis of identified non-fluorescent clones.
Fig. 4: Growth of targeted fluorescent and non-fluorescent (marked with arrow) ES clones.

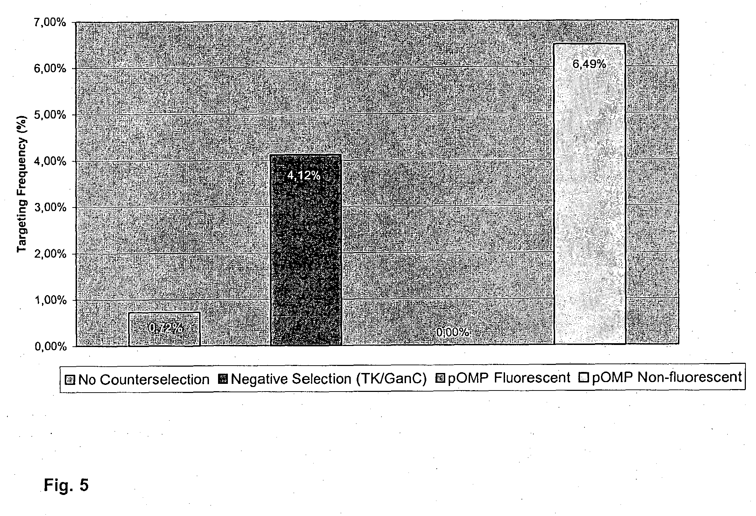

Fig. 5: Enrichment of gene targeting frequency in non-fluorescent ES clones.

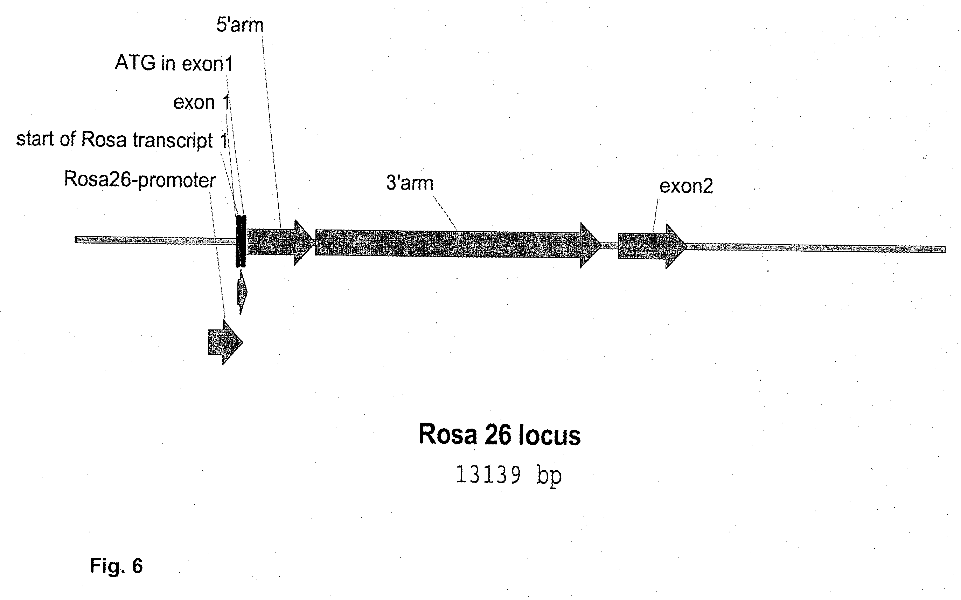

Fig. 6 shows schematically the functional segments of the rosa26 locus.

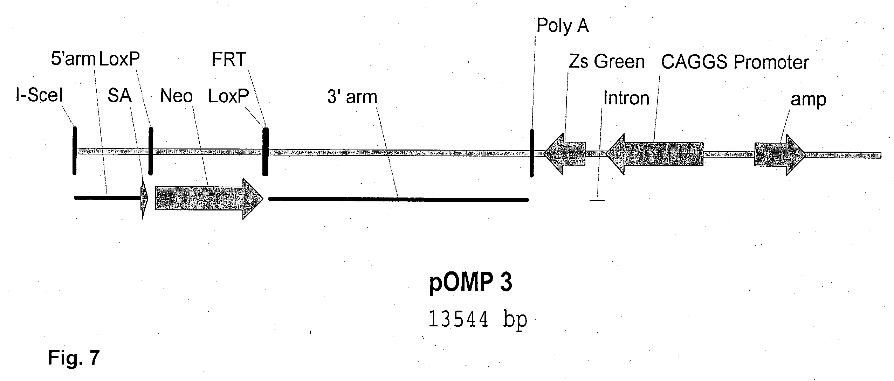

Fig. 7 shows the vector pOMP3.

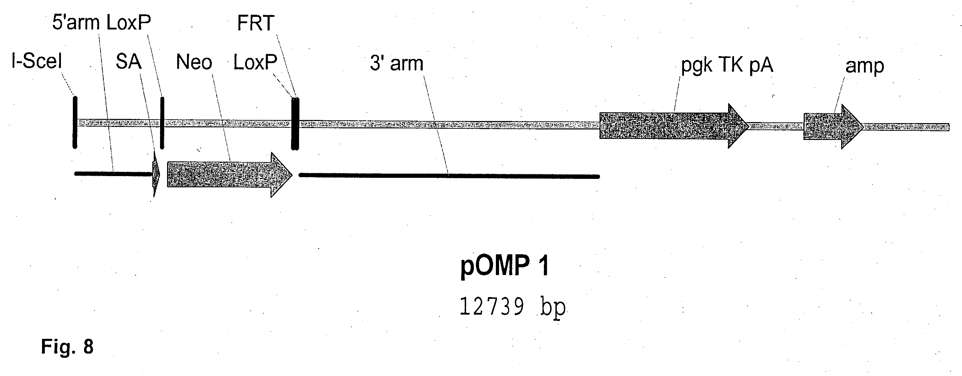

Fig. 8 shows the vector POMP1.

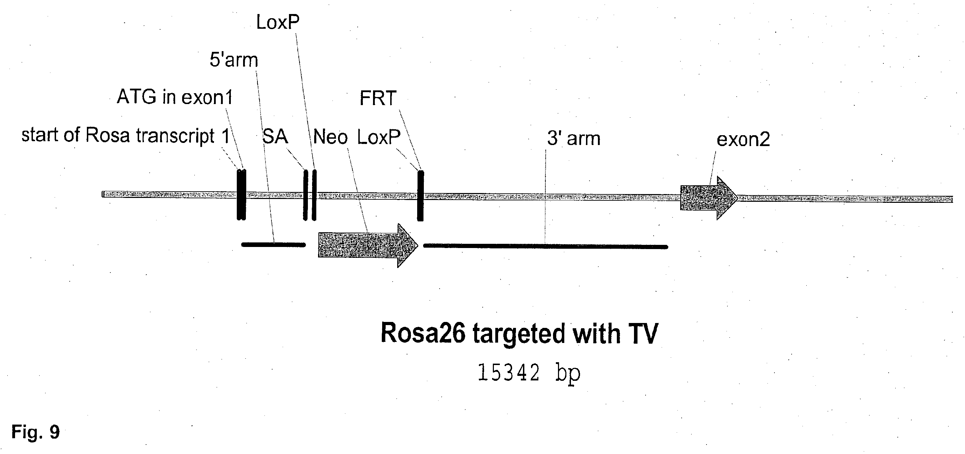

Fig. 9 shows schematically the modified rosa26 locus.

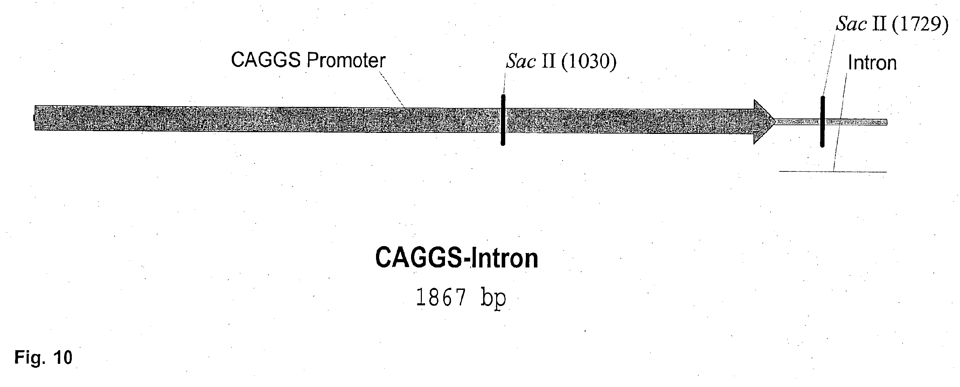

Fig. 10 shows schematically the CAGGS promoter followed by an intron.

### Detailed Description of the Invention

"Targeted vector" or "vector for targeted homologous recombination" according to the present invention is a DNA construct that contains sequences "homologous" to endogenous chromosomal nucleic acid sequences which flank a desired genetic modification. The flanking homologous sequences direct the targeting vector to the specific chromosomal location within a genome by virtue of the homology that exists between the flanking homologous sequences and the corresponding endogenous sequences and introduce the desired genetic modification (i.e. a functional DNA segment) by a process referred to as "homologous recombination".

"Homologous" means two or more nucleic acid sequences that are either identical or similar enough that they are able to hybridize to each other or undergo intermolecular exchange.

A "flanking DNA" is a segment of DNA that is collinear with and adjacent to a particular point of reference.

"Gene targeting" is the modification of an endogenous chromosomal locus by the insertion into, deletion of or replacement of the endogenous sequence via homologous recombination using a targeting vector.

The "homologous targeting cassette" is that part of the targeted vector that carries the desired genetic modification, the flanking homologous sequences and optionally functional sequences such as selectable or detectable markers.

The "expression cassette" is that part of the targeted vector that enables distinction of non-targeted and correctly targeted stably transfected cells by determining its presence or absence in the transfected cellular system. The two cassettes are "connected" through covalent linkage of an arbitrary number of nucleotides.

A "detectable marker" only allows for the detection and subsequent isolation of a transfected cell, whereas a "selectable marker" or "selection marker" provides for a selection advantage of the cell containing said marker over cells devoid of said marker and therewith allows for the enrichment of rare transfected cell expressing the marker from the majority of treated cells in the population.

"Multi-cell organisms" according to the present invention include vertebrates, invertebrates or plants. The "eukaryotic cells", "transgenic tissues" or "transgenic organs" are derived from the multi-cell organisms referred to above. The term "eukaryotic cells" also includes, however, single cell eukaryotic cells. An "ES cell" is an embryonic stem cell.

A "non-human organism" or "non-human mammal" is an organism or mammal that is not normally accepted by the public as being human.

In a preferred embodiment of the invention a vector as defined in (1) above has the following properties: The homologous targeting cassette (A) comprises (A1) a functional DNA segment and a positive selection marker or a functional DNA segment and a second detectable marker different from said first detectable marker, and (A2) two DNA segments homologous to the integration site within the genome of the eukaryotic cells flanking said functional DNA segment and said positive selection marker or said functional DNA segment and said second detectable marker (A1).

It is also preferred that the expression cassette (B) comprises a gene coding for said first detectable marker under the control of a promoter (e.g. constitutive, inducible, etc.) active in eukaryotic cells. Preferably the promoter is a constitutive promoter including, but not limited to, CAGGS (Niwa, H.K. et al., Gene 108(2):193-9 (1991)), CMV (Bi, J.X. et al., Biotechnol. Bioeng. 81(7):848-54 (2003)), c-fos (Bi, J.X. et al., Biotechnol. Bioeng. 81(7):848-54 (2003)), PGK (Adra, C.N. et al., Gene 60:65-74 (1987)), SV40 (Southern, P.J., Berg, P., J. Mol. Appl. Genet. 1:327-341 (1982)), elongation factor 1alpha (eF1alpha), RNA polymerase II (Soriano, P. et al., J. Virol. 65(5):2314-9 (1991)) and TK.

Moreover, it is preferred that said first and/or second detectable marker are a non-toxic, directly or indirectly detectable compound or the marker(s) are gene(s) expressing membrane bound protein(s) or protein(s) with membrane anchoring signal sequence, requiring that such proteins are not expressed in the transfected cell of interest. Such membrane bound proteins can be identified by binding to fluorescence or otherwise labeled detectable antibodies.
Preferably the detectable marker is a compound which is detectable by colorimetric (Lowry method, Bradford reaction (Comassie blue), sulforhodamine B, β-galactosidase (lacZ), placental alkaline phosphatase (PAP)), fluorescence, bioluminescence such as luciferases from phengodid beetles, which can produce green to red bioluminescence Viviani, V.R., Cell Mol. Life Sci. 59(11):1833-50 (2002)) phosphorescence detection methods or the like (for review of probes see (Haughland, R.P., Molecular Probes. Handbook of fluorescent probes and research chemicals, 172-180, 221-229 (1992-1994); Freshney, R.I., Culture of animal cells, 3^{rd} ed., Wiley & Sons, Inc. (1994)). Most preferred is the use of a fluorescence protein/peptide.

According to the invention the expression cassette may be positioned 5' or 3' of a homologous targeting cassette. If the homologous targeting cassette harbors at least two regions of target gene homology which differ in length, the position of the expression cassette is preferably connected with the longer region of homology.

According to the invention the eukaryotic cells are derived from multi-cell organisms selected from vertebrates, including mammals and fish, invertebrates including insects and worms, and plants. The eukaryotic cells can be immortalized cells or primary cells derived from said multi-cell organism. The eukaryotic cells can also be single cell eukaryotic cells.

In a more preferred embodiment the vector of (1) above the functional DNA segment of the targeting cassette is a DNA sequence encoding a gene of interest, or a part of a gene of interest, or is a functional DNA sequence which can be converted into such DNA sequence encoding a gene of interest, or intronic sequence, or is a regulatory functional DNA sequence, including, but not limited to, splice receptor of splice donor or acceptor sequences, and recombinase recognition site(s) (RRS(s)).

The functional DNA segment may further comprise gene expression control elements including, but not limited to, ubiquitous or tissue specific promoter, either constitutive or inducible, a polyadenylation signal, intron sequences, recombinase recognition site(s), enhancer recognition site(s), and matrix attachment region(s) (MAR).
The positive selection marker according to the present invention may be a DNA sequence encoding a protein conferring resistance against cell poison, including, but not limited to, neomycine (Beck, E. et al., Gene 19(3):327-36 (1982)), hygromycine (Bernard, H.U. et al., Exp. Cell Res. 158(1):237-43 (1985); Gordano, T.J., McAllister, W.T., Gene 88(2):285-8 (1990); Santerre, R.F. et al., Gene 30(1-3):147-56 (1984)), puromycine N actyltransferase (*puro*) (de la Luna, S. et al., Gene 62(1):121-6 (1988); de la Luna, S., Ortin, J., Methods Enymol. 216:376-85 (1992)), *E*. *coli* xanthine guanosine phosphoribolsyltransferase (*gpt*) (Spring, K.J. et al., Biochim. Biophys. Acta 1218(2):158-62 (1994)), blasticidin (Kobayashi, K. et al., Agric. Biol. Chem. 55(12):3155-7 (1991); Kimura, M. et al., Mol. Gen. Genet. 242(2):121-9 (1994)), hypoxanthine-guanosine phosphorybosyltransferase (*Hprt*) (Albertini, R.J. et al., Nature 316(6026):369-71 (1985); Lester, S.C. et al., Somatic Cell. Genet. 6(2):241-59 (1980)), herpes simplex virus type 1 thymidine kinase *(hsvTK)* (Borrelli, E. et al., Proc. Natl. Acad. Sci. USA 85(20):7572-6 (1988)), and adenine phosphoribosyltransferase (*aprt*) (Lester, S.C. et al., Somatic Cell. Genet. 6(2):241-59 (1980)) - HPRT, TK and aprt in positive selection schemes require ES cells with mutations in their endogeneous *Hprt* or *TK* loci - or may be a DNA sequence, conferring superior metabolic properties to the cells, as compared to non-transfected (starting) cells, including the utilization of xanthine, adenine, etc.

According to the present invention the first and/or second marker are DNA sequences encoding a protein allowing direct optical detection, such as by detection of fluorescence, chemiluminescence or the like, or indirect optical detection, such as a colorimetric assay or the like, provided that said second detectable marker differs from said first detectable marker in order to allow separate detection. It is particularly preferred that the first and/or second detectable marker peptide is a fluorescence protein including, but not limited to, green fluorescence protein (GFP) and homologous, analogous or color variants thereof, and Reef Coral Fluorescent Proteins (RCFPs) such as AmCyan, ZsGreen, ZsYellow, DsRed, AsRed and HcRed (Clontech, Palo Alto, CA). Particularly preferred are the fluorescent peptides mentioned in the following Table 1 (Labas, Y.A. et al., PNAS 99(7):4256-4261 (2002)):

**Table 1:**

| Summary of family of GFP-like proteins | | | |
|---|---|---|---|
| ***Protein ID*** | ***Original ID*** | ***GenBank accession*** | ***# Genus species*** |
| amajGFP | amFP486 | AF 168421 | *Anemonia majano* |
| dsfrGFP | DsFP483 | AF 168420 | *Discosoma striata* |
| clavGFP | CFP484 | AF 168424 | *Clavularia sp.* |
| GFP | | M 62653 | *Aequora victoria* |
| cgigGFP | | AY 037776 | *Condylactis gigantea* |
| hcriGFP | | AF 420592 | *Heteractis crispa* |
| ptilGFP | | AY 015995 | *Ptilosarcus sp.* |
| rmueGFP | | AY 015996 | *Renilla muelleri* |
| zoanGFP | zFP560 | AF 168422 | *Zoanthus sp.* |
| asulGFP | asFP499 | AF 322221 | *Anemonia sulcata* |
| dis3GFP | | AF 420593 | *Discosoma sp.3* |
| dendGFP | | AF 420591 | *Dendronephthya sp.* |
| mcavGFP | | AY 037769 | *Montastraea cavernosa* |
| rfloGFP | | AY 037772 | *Ricordea florida* |
| scubGFP1 | | AY 037767 | *Scolymia cubensis* |
| scubGFP2 | | AY 037771 | *Scolymia cubensis* |
| zoanYFP | | AF 168423 | *Zoanthus sp.* |
| DsRed | drFP583 | AF 168419 | *Discosoma sp.1* |
| dis2RFP | dsFP593 | AF 272711 | *Discosoma sp.2* |
| zoan2RFP | | AY 059642 | *Zoanthus sp.2* |
| mcavRFP | | AY 037770 | *Montastraea cavernosa* |
| rfloRFP | | AY 037773 | *Ricordea florida* |
| asulCP | asCP | AF 246709 | Anemonia sulcata |
| hcriCP | hcCP | AF 363776 | *Heteracis crispa* |
| cgigCP | cpCP | AF 363775 | *Condylactis gigantea* |
| cpasCP | cpCP | AF 383155 | *Condylactis parsiflora* |
| gtenCP | gtCP | AF 383156 | *Goniopora tenuidens* |

It is moreover preferred that the positive selection marker or the second detectable marker are flanked by RRSs in order to allow removal of the marker by means of a recombinase after the proper recombination product was established.
The flanking homologous DNA segments may have a length of 0.1 to 20 kb, preferably 0.5 to 10 kb. The actual flanking DNA sequences depend on the locus of integration, suitable sequences are those homologous to the Rosa26, HPRT, beta-actin, GAPDH locus or the like of eukaryotic cells.

In a particularly preferred vector of the invention the first detectable marker is ZsGreen and the constitutive promoter is the CAGGS promoter. The targeting cassette in such a vector may contain a positive selection marker as defined above or a second fluorescence marker differing from the first marker. Suitable marker combinations out of the ones mentioned in Table I are known in the art. Reef Coral Fluorescent Proteins (RCFPs) such as AmCyan, ZsGreen, ZsYellow, DsRed, AsRed and HcRed (Clontech, Palo Alto, CA) each emit a distinct wavelength. The corresponding cDNAs were isolated from nonbioluminescent reef corals (class Anthozoa), and have been optimized for bright emission, fast chromophore maturation, and codon optimized for increased expression in mammalian cells. Because of their distinct spectra RCFPs can be used in combination to visualize multiple events simultaneously.

In a most preferred vector the homologous sequences of the targeting cassette are homologous to the mouse Rosa26 locus and the selection marker is a loxP flanked neomycin resistance gene (see bp 1287 to 3233 of SEQ ID NO:6), preferably said vector has the sequence of SEQ ID. NO. 6.

The method (2) of the invention comprises, but is not limited to, the following steps:
1. Generation of a gene targeting construct harbouring one or more expression cassettes coding for non-identical optical markers, e.g. two fluorescence molecules (ZsGreen, AmCyan1 or ZsYellow1 or DsRed2, DsRedExpress or AsRed2, or HcRed1; Clontech, Palo Alto,CA) under control of a constitutive CAGGS promoter (Niwa, H. et al., Gene, 108(2):193-9 (1991)).
2. Gene targeting by homologous recombination in embryonic stem (ES) cells with this vector.
3. Identification of preferentially homologous recombined ES clones harbouring the second detectable marker, not harbouring the first detectable marker.
4. Isolation of such clones by
   (a) manual picking of non-fluorescent clones under fluorescence light emission source;
   (b) limited dilution plating of electroporated ES cells in multi-well cell culture dishes at a pre-determined concentration to enable preferential growth of single clones/well;
      (i) manual sub-culture and molecular analysis of identified non-fluorescent colonies by current protocols;
      (ii) automated sub-culture and PCR analysis of identified non-fluorescent colonies;
   (c) fluorescent activated cell sorting (FACS) of preferentially homologous recombined ES cells expressing or not expressing the detectable fluorescent marker and plating of sorted cells at single cell density in multi-well tissue culture plates.

The method (3) of the invention for preparing transgenic tissues, organs and/or multi-well organisms is exemplified by the following steps:
(a) Generation of genetically modified organisms by
   (aa) ES cells. Such cells can be used to create genetically modified rats or mice by standard blastocyst injection technology (Robertson, E.J. Editor., IRL Press: Oxford, UK, 71-112 (1987)), aggregation (Wood, S.A. et al., Nature 365(64411):87-89 (1993)), tetraploid blastocyst injection (Wang, Z. Q et al., Mech. Dev. 62(2):137-45 (1997)), nuclear transfer and cloning (Wakayama, T. et al., Proc. Natl. Acad. Sci. USA 96(26):14984-9 (1999)), ES cells derived from other organism such as rabbits (Wang, Z. Q et al., Mech. Dev. 62(2):137-45 (1997); Schoonjans, L. et al., Mol. Reprod. Dev. 45(4):439-43 (1996)), chickens (Pain, B. et al., Development 122(8):2339-48 (1996)), or other species;
   (bb) modified protoplasts to generate genetically modified plants;
   (cc) nuclear transfer from modified eukaryotic cells to oocytes to generate cloned organisms with modified allele (Wakayama, T. et al., Proc. Natl. Acad. Sci. USA 96(26):14984-9 (1999); Baguisi, A. et al., Nat. Biotechnol. 17(5):456-61 (1999); Wilmut, I.L. et al., Nature 385(6619):810-3 (1997); Wilmut, I.L. et al., Reprod. Fertil. Dev. 10(7-8):639-43 (1998); Wakayama, T., Protein, Nucleic Acid Enzyme 45(13 Suppl.):2005-14 (2000); Wakayama, T. et al., Nature 394(6691):369-74 (1998); Rideaut, W.M., Nat. Genet. 24(2):109-10(2000); Campbell, K.H. et al., Nature, 380(6569):64-6 (1996));
   (dd) cell-fusion to transfer the modified allele to another cell, including transfer of engineered chromosome(s), and uses of such cell(s) to generate organisms carrying the modified allele or engineered chromosome(s) (Kuroiwa, Y. et al., Nat. Biotechnol. 18(10):1086-90 (2000)).
(b) Cloning: The techniques used to construct DNA vectors described herein are standard molecular biology techniques well known to the skilled molecular biologist (Sambrook, J. Fritsch, E.F.a.M., Molecular Cloning: a Laboratory Maunal, 2^{nd} ed., vols. 1, 2 and 3, Cold Spring Harbor, NY, USA, Wiley Interscience, NY (1989)).
(c) Gene modifications including
   (aa) deletion of coding sequences, gene segments, or regulatory elements;
   (bb) alterations(s) of coding sequence, gene segments, or regulatory elements including substitutions, additions, and fusions (e.g. epitope tags or creation of bifunctional proteins);
   (cc) insertion of new coding regions, gene segments, or regulatory elements, such as those for selectable marker genes or reporter genes or putting new genes under endogenous transcriptional control;
   (dd) creation of conditional alleles, e.g. by introduction of loxP sites flanking the region to be excised by Cre recombinase (Abremski, K., Hoess, R., J. Biol. Chem. 259(3):1509-14 (1984) or FRT sites flanking the region to be excised by FLP recombinase (Andrews, B.J. et al., Cell 40(4):795-803 (1985); Meyer-Leon, L. et al., Cold Spring Harb. Symp. Quant. Biol. 49:797-804 (1984)); or
   (ee) replacement of coding sequences or gene segments from one species with orthologous coding sequences from a different species, e.g. replacing a murine genetic locus with the orthologous human genetic locus to engineer a mouse where that particular locus has been 'humanized'.
(d) Introduction of vectors into eukaryotic cells: Using standard methodology, such as transfection mediated by calcium phosphate, lipids, or electroporation (Sambrook, J. Fritsch, E.F.a.M., Molecular Cloning: a Laboratory Maunal, 2^{nd} ed., vols. 1, 2 and 3, Cold Spring Harbor, NY, USA, Wiley Interscience, NY (1989)).
(e) Selection: By exposure to selection agents, depending on the selectable marker gene that has been engineered into the vector. If the selectable marker is the neomycin phosphotransferase (neo) gene (Beck, E. et al., Gene 19(3):327-36 (1982)) then cells that have taken up the vector can be selected in G418-containing media; cells that do not have the vector will die whereas cells that have taken up the vector will survive (Freshney, R.I., Culture of animal cells, 3^{rd} ed., Wiley & Sons, Inc. (1994)). Other suitable selectable markers include any drug that has activity in eukaryotic cells, such as hygromycin B Santerre, R.F. et al., Gene 30(1-3):147-56 (1984); Bernard, H.U. et al., Exp. Cell. Res. 158(1):237-43); Giordano, T.J., McAllister, W.T., Gene 88(2):285-8 (1990)), Blasticidin (Izumi, M. et al., Exp. Cell. Res. 197(2):229-33 (1991)) and other which are familiar to those skilled in the art.

The invention is further described by reference to the following examples, which is, however, not to be construed so as to limit the invention.

### Example

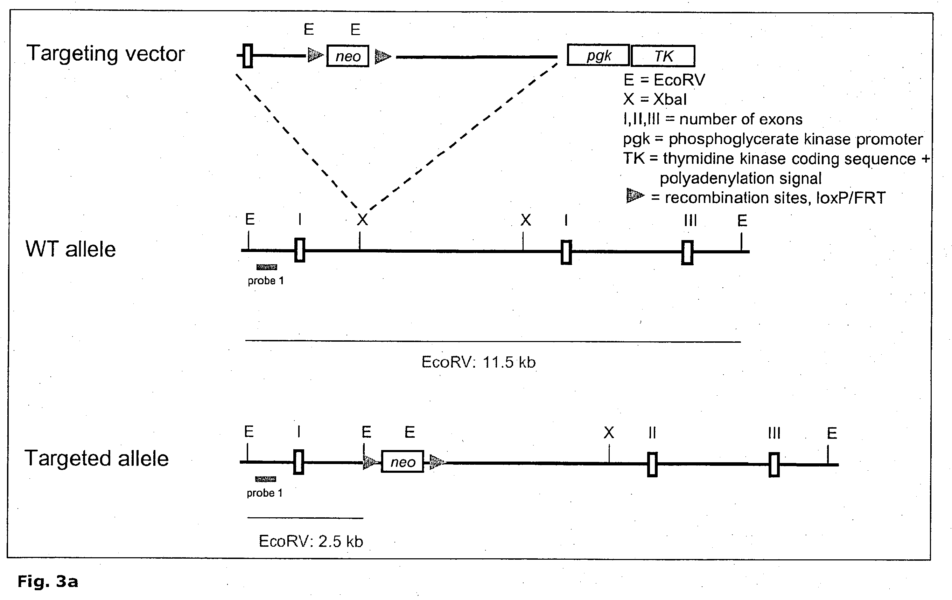

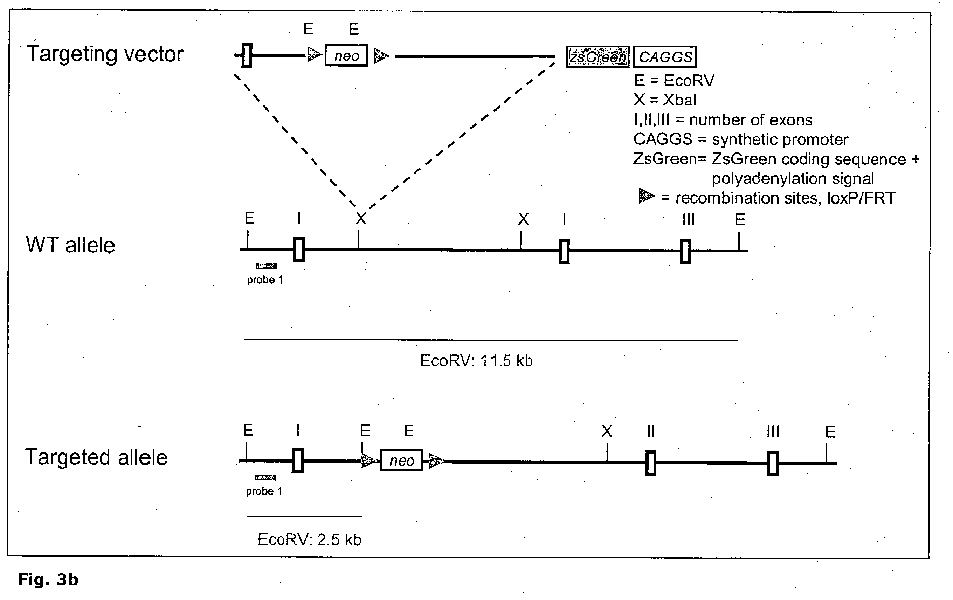

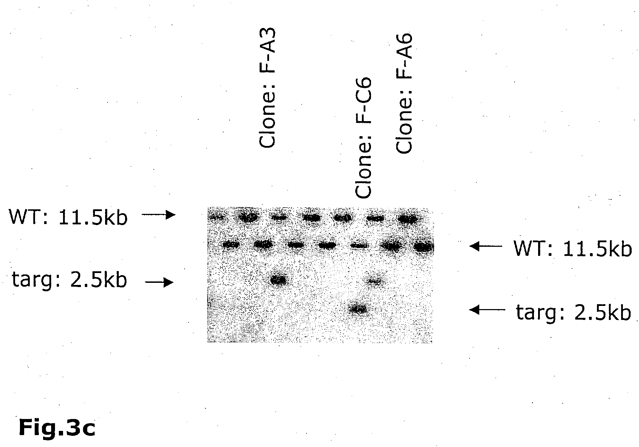

As example for optical identification of ES clones, the fluorescent molecule ZsGreen gene (Clontech) was chosen. The Rosa locus of the mouse (Fig.6, SEQ ID NO:3) was chosen for homologous recombination. The targeting strategy is outlined in Figures 3A, B and C.
1. Rosa Targeting Vector: A 129 Sv/Ev-BAC library (Incyte Genomics) was screened with a probe against *exon2* of the *Rosa26* locus (amplified from mouse genomic DNA using Rscreen1s (GACAGGACAGTGCTTGTTTAAGG; SEQ ID NO:1) and Rscreen1as (TGACTACACAATATTGCTCGCAC; SEQ ID NO:2)). PCR conditions were as follows: 95°C, 2 min, followed by 30 cycles: 95°C; 30 sec; 60°C, 30 sec; 72°C, 30 sec; 72°C, 7 min; followed by 20°C, 2 min. Out of the identified BAC clone a 11 kb EcoRV subfragment was inserted into the HindII site of pBS. Two fragments, a 1 kb SacII/XbaI fragment (SEQ ID NO:4) and a 4 kb XbaI-fragment (SEQ ID NO:5) were used as homology arms and inserted into a vector consisting of a FRT-flanked neomycin resistance gene (unpublished) and a PGK-TK-pA expression cassette for negative selection.
2. pOMP1 and pOMP3 vector Construction: For construction of pOMP1 (Fig. 8 and SEQ ID NO:7), the FRT-flanked neomycin resistance gene was replaced by a loxP-flanked neomycin resistance gene (SEQ ID NO:9). The ZsGreen gene was placed under the control of the CAGGS promoter (SEQ ID NO:11) followed by a synthetic intron (pMultilink CAGGS-zsGreen; Fig. 10, SEQ ID NO:10). This expression cassette was used for a replacement of PGK-TK-pA of pOMP1 resulting in pOMP3 (Fig. 7 and SEQ ID NO:6). pOMP1 and pOMP3 were used for the targeting experiments (Figures 3A, 3B).
3. Introduction of pOMP vectors into ES cells
The ES cell line Art4.12 (Eggan, K. et al., Nat. Biotechnol., 20(5):p. 455-9 (2002)) was grown on mitotically inactivated feeder layer (Mitomycin C (Sigma M-0503)) comprised of mouse embryonic fibroblasts in medium composed of 1x DMEM high Glucose (Invitrogen 41965-062), 4 mM Glutamin (Invitrogen 25030-024), 1x Non Essential Amino Acids (Invitrogen 11140-035) 1mM Sodium Pyruvat (Invitrogen 11360-039), 20 mM Hepes (Invitrogen 15630-056), 0.1 mM β-Mercaptoethanol (Invitrogen 31350-010), 2x10⁶ µ/l Leukemia Inhibitory Factor (Chemicon ESG 1107) and 15% fetal bovine serum (PAN 1302-P220821).
Vectors pOMP1 and pPOMP 3 were introduced into the cells by electroporation using a Gene Pulser with Capacitance Extender (Biorad).
Rapidly growing cells were used on the first day following the last passage. Upon trypsinization (Invitrogen 25200-056) cells were resuspended in PBS (Invitrogen 20012-019) and preplated for 25 min on gelatinized 10 cm plates to remove unwanted feeder cells. The supernatant was harvested, ES cells were washed once in PBS and counted (Neubauer hemocytometer). 10⁷ cells were mixed with 30 µg of I-Sce linearized vector in 800 µl of transfection buffer (20 mM Hepes, 137 mM NaCl, 15 mM KCI, 0.7 mM Na₂HPO₄, 6 mM Glucose 0.1 mM β-Mercaptoethanol in H₂O) and electroporated using a Biorad Gene Pulser with Capacitance Extender set on 240 V and 500 µF. Electroporated cells were seeded at a density 0.25*10⁷ cells per 10 cm tissue culture dish onto a previously prepared layer of neomycin-resistant inactivated mouse embryonic fibroblasts.
4. Manual identification of ES cells containing a targeted disruption of the Rosa locus
48 h after electroporation, the medium was replaced on all dishes by medium containing 250 µg/ml Geneticin (Invitrogen 10131-019) for positive selection of G418 resistant ES clones.
5 day after electroporation, ½ of the dishes containing ES cells electroporated with vector pOMP1 were negatively selected by addition of 2 uM Gancyclovir (Cymeven®, Roche)
On day 8 after electroporation ES colonies were isolated as follows:
Medium was replaced by PBS and the culture dishes were placed on the stage of a binocular (Nikon SMZ-2B). Using low magnification (25 x) individual ES clones of undifferentiated appearance were removed from the surface of the culture dish by suction into the tip of a 20 ul pipette (Eppendorf). The harvested clones were placed in individual wells of 96 well plates containing 30 µl of 2.5% Trypsin (Invitrogen). After disruption of clones by pipetting with a multichannel pipette (Eppendorf), cells were seeded onto feeder containing 96 well plates with pre-equilibrated complete ES-medium.
For pOMP1 positive selected clones (Geneticin) and positive/negative selected clones (Geneticin and Gancyclovir) were harvested as described above.
For pOMP3 only positive selected clones were isolated either at random as described above, or the ES clones were placed under a binocular (Leica MZFL III) with fluorescent light source (GFP3 Filters set) and bright-field illumination. At dim light, clearly identifiable non-fluorescent ES clones (Figure 4) were isolated as described above.
Cells were grown for 3 days with daily medium changes and then splitted 1 : 2 on gelatinized (Sigma G-1890) 96 well plates. 3 days after splitting cells were lysed, genomic DNA was prepared and analysed by Southern blot.
5. Identification of non-fluorescent transfected cells by plate-reader analysis
Electroporated cells were seeded at a density of 1 cell per well in a 96 multi-well plate, coated with a previously prepared layer of neomycin-resistant inactivated mouse embryonic fibroblasts.
48 h after electroporation, the medium was replaced on all dishes by medium containing 250 µg/ml Geneticin (Invitrogen 10131-019) for positive selection of G418 resistant ES clones.
5 days upon transfection individual wells were scored by plate-reader analysis
a) by O.D. measurement for the presence of single ES clones
b) by fluorescence measurement (GFP3 filter set; Leica, Bensheim, Germany) for the location of non-fluorescent clones.
Identified non-fluorescent ES clones were expanded and taken to molecular analysis by Southern blotting (see Fig. 3a, 3b) according to standard procedures.
6. FACS analysis of transfected cells and single cell plating
24h after electroporation, cells could be trypsinized at a single cell level.
Cells were sorted in multiple rounds by FACS analysis for the presence of the second and absence of the first detectable marker. Sorted single cells were subsequently plated into single entities (individual well of a multi-well dish), further cultivated and taken to molecular analysis as described above.
7. Results
(a) Effect of fluorescence on ES cell growth (Table 2)
Expression of ZsGreen (pOMP3) did not negatively influence ES cell colony formation. The mean number of resistant ES colonies per 1x10⁷ ES cells transfected with the ZsGreen expression vector pOMP3 is similar to the number of ES colonies transfected with pOMP1 (Table 2).

**Table 2:**

| Fluorescence does not negatively effect clonal ES cell growth | | | |
|---|---|---|---|
| | 2^{nd} marker gene | 2^{nd} Selection | # clones per 1 x 10⁷ transfected cells |
| pOMP1 | None | none | 3500 |
| POMP1 | Thymidine Kinase | Gancyclovir | 950 |
| pOMP3 | ZsGreen | none | 3366 |

(b) Selective isolation of non-fluorescent ES colonies. (Table 3) 15,5% of all colonies in the pOMP3 gene targeting experiment were non-fluorescent. Isolation of non-fluorescent ES colonies under fluorescent light-source as described above, led to significant enrichment of such ES colonies. By this method 99,3% (all but 1 clone) were non-fluorescent when monitored upon isolation.

**Table 3:**

| Enrichment of isolation of non-fluorescent ES colonies in pOMP3 experiments. | | |
|---|---|---|
| | | Isolated (%) |
| Random isolation | Fluorescent | 84,5 |
| Random isolation | Non-fluorescent | 15,5 |
| Selective isolation | Fluorescent | 0,7 |
| Selective isolation | Non-fluorescent | 99,3 |

(c) HR frequency in pOMP targeting experiments
The analysis of non-fluorescent ES cells for HR events in the case of pOMP3 targeting resulted in a targeting frequency of 6,49% +/- 0,5% in 3 independent experiments (Table 4).
This is a 9fold higher frequency if compared to targeting of pOMP1 without counterselection. Compared to pOMP1 gene targeting plus counterselection with Gancyclovir (4,12% HR), optical isolation was 63,5% more efficient.

**Table 4:**

| Enrichment of gene targeting frequency in non-fluorescent ES clones. (see also Fig. 5) | | | |
|---|---|---|---|
| Construct | Selection | HR - clones | HR frequency |
| | | | (%) |
| pOMP1 | None | 5/705 | 0,72 |
| pOMP1 | TK/GanC | 7/170 | 4,12 |
| POMP3 | fluorescent | 0 | 0 |
| POMP3 | Non-fluorescent | 15/239 | 6,49 |

## Claims

1. A vector for targeted homologous recombination of eukaryotic cells comprising
(A) a homologous targeting cassette harbouring a functional DNA segment and
(B) an expression cassette harbouring a DNA sequence coding for a first detectable marker, said expression cassette (B) being connected with said homologous targeting cassette (A) so as to allow distinction between targeted and non-targeted cells.

2. The vector of claim 1, wherein
(i) the homologous targeting cassette (A) comprises (A1) a functional DNA segment and a positive selection marker or a functional DNA segment and a second detectable marker different from said first detectable marker, and (A2) two DNA segments homologous to the integration site within the genome of the eukaryotic cells flanking (A1); and/or
(ii) the expression cassette (B) comprises a gene coding for said first detectable marker under the control of a promoter active in eukaryotic cells, preferably the promoter is a constitutive promoter; and/or
(iii) said first and/or second detectable marker is a non-toxic, directly or indirectly detectable compound, preferably is a compound being detectable by colorimetric, fluorescence, chemiluminescence, phosphorescence detection methods or the like, most preferably is a fluorescent peptide; and/or
(iv) the expression cassette (B) is positioned 5' or 3' relative to the homologous targeting cassette; and/or
(v) the eukaryotic cells are derived from multi-cell organisms selected from vertebrates, including mammals and fish, invertebrates including insects and worms, and plants, and can be immortalized or primary cells.

3. The vector of claim 1 or 2, wherein
(i) the functional DNA segment of the targeting cassette (A) is a DNA sequence encoding a gene of interest, or is a functional DNA sequence which can be converted into such DNA sequence encoding a gene of interest, or intronic sequence, or is a regulatory functional DNA sequence, including, but not limited to, splice receptor of splice donor or acceptor sequences, and recombinase recognition site(s);
(ii) the functional DNA segment may further comprise gene expression control elements including, but not limited to, ubiquitous or tissue specific promoter, either constitutive or inducible, a polyadenylation signal, intron sequences, recombinase recognition site(s) (RRS), enhancer recognition site(s), and matrix attachment region(s) (MAR);
(iii) the positive selection marker is a DNA sequence encoding a protein conferring resistance against cell poison, including, but not limited to, neomycine, hygromycine, puromycine, histidinol and bleomycine, is a DNA sequence, conferring superior metabolic properties to the cells, including the utilization of xanthine, adenine etc., or the like;
(iv) the first and/or second marker are DNA sequences encoding a protein allowing direct optical detection, such as by detection of fluorescence, chemiluminescence or the like, or indirect optical detection, such as a colorimetric assay or the like, provided that said second detectable marker differs from said first detectable marker in order to allow separate detection;
(v) the positive selection marker and the second detectable marker are flanked by one or more RRSs; and/or
(vi) the flanking homologous DNA segments have a length of 0.1 to 20 kb, preferably 0.5 to 10 kb; and/or
(vii) the flanking DNA sequences are homologous to the Rosa26, HPRT, beta-actin, GAPDH locus of the eukaryotic cells.

4. The vector of claim 2 or 3, wherein the first and/or second detectable marker peptide is a fluorescence protein including, but not limited to, green fluorescence protein (GFP) and modifications thereof, the constitutive promoter of the expression cassette is selected from GAGGS, CMV, PGK, TK, preferably the fluorescence protein is ZsGreen and the constitutive promoter is the CAGGS promoter.

5. The vector of claim 4, wherein the homologous sequences are homologous to the mouse Rosa26 locus, and preferably said vector has the sequence of SEQ ID. NO. 6.

6. A method for homologous recombination of eukaryotic cells comprising
(a) transfecting cells with a homologous targeting vector as defined in claims 1 to 5 allowing distinction between targeted and non-targeted cells;
(b) identifying and/or isolating cells containing the positive selection marker or the second detectable marker; and
(c) identifying and isolating cells as homologous recombinants by the absence of the first detectable marker.

7. The method of claim 6 which further comprises
(d) plating of transfected cells into standard tissue culture vessels; and/or
(e) limited dilution plating of tranfected cells into multiwell plates, including 96well, 384 well and 1536 well plates, in order to allow preferential growth and analysis of single clones in individual wells/plate, and optionally
subsequent detection and isolation of transfected cells plated as described above by manually or automatically selecting for the absence of the first detectable marker and/or the presence of the second detectable marker.

8. The method of claim 7 wherein the presence of the second and/or absence of the first marker is analyzed by fluorescence activated cell sorting (FACS) mechanism, whereby the isolated sorted cells are re-plated as described in claim 7.

9. The method of claims 6 to 8, wherein the cells are derived from vertebrate, invertebrates or plants, more preferably the vertebrate cells are derived from a mammal such as rodents (mouse, rat, etc.), or a fish such as zebrafish; and/or are primary cell or immortalized cells; most preferably the vertebrate cells are mammalian embryonic stem (ES) cells, most preferably the mammalian ES cells are mouse ES cells.

10. A method for preparing transgenic tissues, organs and/or multi-cell organisms which comprises utilizing the method as defined in claims 6 to 9.

11. The method of claim 10, wherein the transgenic multi-cell organism is a non-human mammal, most preferably mouse, and said method comprises modifying an ES cell as defined in claims 6 to 9.

12. The method of claim 11 which further comprises one or more of the steps
- injecting isolated ES cells in or aggregating isolated ES cells with diploid and/or multiploid preimplantation embryos, preferably blastocysts, or injecting in/fusion of nuclei from ES cells with enucleated non-fertilized eggs (nuclear transfer); and/or generating transgenic non-human embryos and/or animals.


SEQUENCE LISTING

<110> Artemis Pharmaceuticals GmbH

<120> Automated Gene-Targeting Using Non-Toxic Detectable
      Markers

<130> 023502ep JH/BM

<140>
<141>

<160> 12

<170> PatentIn Ver. 2.1

<210> 1
<211> 23
<212> DNA
<213> Artificial Sequence

<220>
<223> Description of Artificial Sequence: Primer
      Rsceen1s

<400> 1
gacaggacag tgcttgttta agg                                         23


<210> 2
<211> 23
<212> DNA
<213> Artificial Sequence

<220>
<223> Description of Artificial Sequence: Primer
      Rscreen1as

<400> 2
tgactacaca atattgctcg cac                                         23


<210> 3
<211> 13139
<212> DNA
<213> Mus musculus

<220>
<223> Description: Rosa 26 locus sequence

<400> 3
aagcttctca cgtagcaacc agagctccag agccagcagc tgctgccgcc ttgtatactc 60
actcctgtga tccaacacag gagcaacctt ttctttaccc cacccccact tcttaacaca 120
cttttttttg gggggggggg gggaacaagt gctccatgct ggaaggattg gaactatgct 180
tttagaaagg aacaatccta aggtcacttt taaattgagg tctttgattt gaaaatcaac 240
aaataccaaa ttccaaatat tcgttttaat taaaccagca atgtggatat aagcattaag 300
ttttagtttt aaaaaggtca attttccaaa cattcagcaa tcatatttaa atttacagct 360
aggaacaaga gccttgggtc atgtcctacc aaagaacata actcaatatt ctacacatga 420
caatctgaat aaccttaaag cctctaatcc cataacaggc cacaaatttt ggacagagaa 480
ctaatgatcc tcctgagaaa actggaagaa atccagggaa aagaaattcc tgtgtcctcc 540
aaactcagaa atctctaatt atgtcagtat tctctgcttt agtcctaggt cagattgcac 600

acatctaaaa taacctctta aagttttcct cctagcgacc taaaccatta ttaatatcaa 660
attaaccatc aaaacacttt cctctcaata tgctgcacac aaacctcctc ctggaacctc 720
ctccatctgg atcctcccca atcaaaagta taggtattta acatataagc aaggaagtaa 780
tgtaaacatg accttggtca caaatatgtc atctaaaaac aatttagtca aggtatggag 840
gaaattcgag aacctgaatc tttttaagta ttttgagcac aggaacaatt ggcaaaagga 900
atccaggtat agacaaaacc cagagcccag agctctgggc gaaaaatgag ttgctggtga 960
agacgttaca caagtaacat gagaaagcag aaaatgcagg tcatccacgc acccctgacc 1020
caggccagca gggcgggctg cagcatcagt acacaggaga aagatcctta ttcctaagaa 1080
tgagaaaggc aaaggcgccc gatagaataa attagcatag aaggggcttt cccaggagtt 1140
aaaactttcc ttctgagcga ttacctacta aaaccagggc ttttgcccac taccatttac 1200
ctaggatctt ggcttgcacg gattcatagg ggcatatccc tccccctctt ctttagagtc 1260
gttcttaaaa gatcgctctc cacgccctag gcagggaaaa cgacaaaatc tggctcaatt 1320
ccaggctaga accctacaaa ttcaacaggg atatcgcaag gatactgggg catacgccac 1380
agggagtcca agaatgtgag gtgggggtgg cgaaggtaat gtctttggtg tgggaaaagc 1440
agcagccatc tgagatagga actggaaaac cagaggagag gcgttcagga agattatgga 1500
ggggaggact gggcccccac gagcgaccag agttgtcaca aggccgcaag aacaggggag 1560
gtggggggct cagggacaga aaaaaaagta tgtgtatttt gagagcaggg ttgggaggcc 1620
tctcctgaaa agggtataaa cgtggagtag gcaataccca ggcaaaaagg ggagaccaga 1680
gtagggggag gggaagagtc ctgacccagg gaagacatta aaaaggtagt ggggtcgact 1740
agatgaagga gagcctttct ctctgggcaa gagcggtgca atggtgtgta aaggtagctg 1800
agaagacgaa aagggcaagc atcttcctgc taccaggctg ggaggcccca ggcccacgac 1860
cccgaggaga gggaacgcag ggagactgag gtgacccttc tttccccgg ggcccggtcg 1920
tgtggttcgg tgtctctttt ctgttggacc cttaccttga cccaggcgct gccggggcct 1980
gggcccgggc tgcggcgcac ggcactcccg ggaggcagcg agactcgagt taggcccaac 2040
gcggcgccac ggcgtttcct ggccgggaat ggcccgtacc cgtgaggtgg gggtgggggg 2100
cagaaaaggc ggagcgagcc cgagcgggga gggggagggc caggggcgga gggggccggc 2160
actactgtgt tggcggactg gcgggactag ggctgcgtga gtctctgagc gcaggcgggc 2220
ggcggccgcc cctcccccgg cggcggcagc ggcggcagcg gcggcagctc actcagcccg 2280
ctgcccgagc ggaaacgcca ctgaccgcac ggggattccc agtgccggcg ccaggggcac 2340
gcgggacacg ccccctcccg ccgcgccatt ggcctctccg cccaccgccc cacacttatt 2400
ggccggtgcg ccgccaatca gcggaggctg ccggggccgc ctaaagaaga ggctgtgctt 2460
tggggctccg gctcctcaga gagcctcggc taggtagggg atcgggactc tggcgggagg 2520
gcggcttggt gcgtttgcgg ggatgggcgg ccgcggcagg ccctccgagc gtggtggagc 2580
cgttctgtga gacagccggg tacgagtcgt gacgctggaa ggggcaagcg ggtggtgggc 2640
aggaatgcgg tccgccctgc agcaaccgga gggggaggga gaaggagcgg gaaaagtctc 2700
caccggacgc ggccatggct cggggggggg ggggcagcgg aggagcgctt ccggccgacg 2760
tctcgtcgct gattggcttc ttttcctccc gccgtgtgtg aaaacacaaa tggcgtgttt 2820
tggttggcgt aaggcgcctg tcagttaacg gcagccggag tgcgcagccg ccggcagcct 2880
cgctctgccc actgggtggg gcgggaggta ggtggggtga ggcgagctgg acgtgcgggc 2940
gcggtcggcc tctggcgggg cgggggaggg gagggagggt cagcgaaagt agctcgcgcg 3000
cgagcggccg cccaccctcc ccttcctctg ggggagtcgt tttacccgcc gccggccggg 3060
cctcgtcgtc tgattggctc tcggggccca gaaaactggc ccttgccatt ggctcgtgtt 3120
cgtgcaagtt gagtccatcc gccggccagc gggggcggcg aggaggcgct cccaggttcc 3180
ggccctcccc tcggccccgc gccgcagagt ctggccgcgc gcccctgcgc aacgtggcag 3240
gaagcgcgcg ctgggggcgg ggacgggcag tagggctgag cggctgcggg gcgggtgcaa 3300
gcacgtttcc gacttgagtt gcctcaagag gggcgtgctg agccagacct ccatcgcgca 3360
ctccggggag tggagggaag gagcgagggc tcagttgggc tgttttggag gcaggaagca 3420
cttgctctcc caaagtcgct ctgagttgtt atcagtaagg gagctgcagt ggagtaggcg 3480
gggagaaggc cgcacccttc tccggagggg ggaggggagt gttgcaatac ctttctggga 3540
gttctctgct gcctcctggc ttctgaggac cgccctgggc ctgggagaat cccttccccc 3600
tcttccctcg tgatctgcaa ctccagtctt tctagaagat gggcgggagt cttctggcca 3660
ggcttaaagg ctaacctggt gtgtgggcgt tgtcctgcag gggaattgaa caggtgtaaa 3720
attggaggga caagacttcc cacagatttt cggttttgtc gggaagtttt ttaatagggg 3780
caaataagga aaatgggagg ataggtagtc atctggggtt ttatgcagca aaactacagg 3840
ttattattgc ttgtgatccg cctcggagta ttttccatcg aggtagatta aagacatgct 3900
cacccgagtt ttatactctc ctgcttgaga tccttactac agtatgaaat tacagtgtcg 3960
cgagttagac tatgtaagca gaattttaat catttttaaa gagcccagta cttcatatcc 4020
atttctcccg ctccttctgc agccttatca aaaggtattt tagaacactc attttagccc 4080
cattttcatt tattatactg gcttatccaa cccctagaca gagcattggc attttccctt 4140
tcctgatctt agaagtctga tgactcatga aaccagacag attagttaca tacaccacaa 4200
atcgaggctg tagctggggc ctcaacactg cagttctttt ataactcctt agtacacttt 4260

ttgttgatcc tttgccttga tccttaattt tcagtgtcta tcacctctcc cgtcagtggt 4320
gttccacatt tgggcctatt ctcagtccag ggagttttac aacaatagat gtattgagaa 4380
tccaacctaa agcttaactt tccactccca tgaatgcctc tctccttttt ctccatttat 4440
aaactgagct attaaccatt aatggttcca ggtggatgtc tcctccccat attacctgat 4500
gtatcttaca tattgccagg ctgatatttt aagacattaa aaggtatatt tcattattga 4560
gccacatggt attgattact gcttactaaa attttgtcat tgtacacatc tgtaaaaggt 4620
ggttcctttt ggaatgcaaa gttcaggtgt ttgttgtctt tcctgaccta aggtcttgtg 4680
agcttgtatt ttttctattt aagcagtgct ttctcttgga ctggcttgac tcatggcatt 4740
ctacacgtta ttgctggtct aaatgtgatt ttgccaagct tcttcaggac ctataatttt 4800
gcttgacttg tagccaaaca caagtaaaat gattaagcaa caaatgtatt tgtgaagctt 4860
ggtttttagg ttgttgtgtt gtgtgtgctt gtgctctata ataatactat ccaggggctg 4920
gagaggtggc tcggagttca agagcacaga ctgctcttcc agaagtcctg agttcaattc 4980
ccagcaacca catggtggct cacaaccatc tgtaatggga tctgatgccc tcttctggtg 5040
tgtctgaaga ccacaagtgt attcacatta aataaataaa tcctccttct tcttcttttt 5100
ttttttttta aagagaatac tgtctccagt agaatttact gaagtaatga aatactttgt 5160
gtttgttcca atatggtagc caataatcaa attactcttt aagcactgga aatgttacca 5220
aggaactaat ttttatttga agtgtaactg tggacagagg agccataact gcagacttgt 5280
gggatacaga agaccaatgc agactttaat gtcttttctc ttacactaag caataaagaa 5340
ataaaaattg aacttctagt atcctatttg tttaaactgc tagctttact taacttttgt 5400
gcttcatcta tacaaagctg aaagctaagt ctgcagccat tactaaacat gaaagcaagt 5460
aatgataatt ttggatttca aaaatgtagg gccagagttt agccagccag tggtggtgct 5520
tgcctttatg cctttaatcc cagcactctg gaggcagaga caggcagatc tctgagtttg 5580
agcccagcct ggtctacaca tcaagttcta tctaggatag ccaggaatac acacagaaac 5640
cctgttgggg aggggggctc tgagatttca taaaattata attgaagcat tccctaatga 5700
gccactatgg atgtggctaa atccgtctac ctttctgatg agatttgggt attatttttt 5760
ctgtctctgc tgttggttgg gtcttttgac actgtgggct ttctttaaag cctccttcct 5820
gccatgtggt ctcttgtttg ctactaactt cccatggctt aaatggcatg gctttttgcc 5880
ttctaagggc agctgctgag atttgcagcc tgatttccag ggtggggttg ggaaatcttt 5940
caaacactaa aattgtcctt taattttttt tttaaaaaat gggttatata ataaacctca 6000
taaaatagtt atgaggagtg aggtggacta atattaaatg agtccctccc ctataaaaga 6060
gctattaagg ctttttgtct tatacttaac ttttttttta aatgtggtat ctttagaacc 6120
aagggtctta gagttttagt atacagaaac tgttgcatcg cttaatcaga ttttctagtt 6180
tcaaatccag agaatccaaa ttcttcacag ccaaagtcaa attaagaatt tctgactttt 6240
aatgttaatt tgcttactgt gaatataaaa atgatagctt ttcctgaggc agggtctcac 6300
tatgtatctc tgcctgatct gcaacaagat atgtagacta aagttctgcc tgcttttgtc 6360
tcctgaatac taaggttaaa atgtagtaat acttttggaa cttgcaggtc agattctttt 6420
ataggggaca cactaaggga gcttgggtga tagttggtaa aatgtgtttc aagtgatgaa 6480
aacttgaatt attatcaccg caacctactt tttaaaaaaa aaagccaggc ctgttagagc 6540
atgcttaagg gatccctagg acttgctgag cacacaagag tagttacttg gcaggctcct 6600
ggtgagagca tatttcaaaa aacaaggcag acaaccaaga aactacagtt aaggttacct 6660
gtctttaaac catctgcata tacacaggga tattaaaata ttccaaataa tatttcattc 6720
aagttttccc ccatcaaatt gggacatgga tttctccggt gaataggcag agttggaaac 6780
taaacaaatg ttggttttgt gatttgtgaa attgttttca agtgatagtt aaagcccatg 6840
agatacagaa caaagctgct atttcgaggt ctcttggttt atactcagaa gcacttcttt 6900
gggtttccct gcactatcct gatcatgtgc taggcctacc ttaggctgat tgttgttcaa 6960
ataaacttaa gtttcctgtc aggtgatgtc atatgatttc atatatcaag gcaaaacatg 7020
ttatatatgt taaacatttg tacttaatgt gaaagttagg tctttgtggg tttgattttt 7080
aattttcaaa acctgagcta aataagtcat ttttacatgt cttacatttg gtggaattgt 7140
ataattgtgg tttgcaggca agactctctg acctagtaac cctacctata gagcactttg 7200
ctgggtcaca agtctaggag tcaagcattt caccttgaag ttgagacgtt ttgttagtgt 7260
atactagttt atatgttgga ggacatgttt atccagaaga tattcaggac tatttttgac 7320
tgggctaagg aattgattct gattagcact gttagtgagc attgagtggc ctttaggctt 7380
gaattggagt cacttgtata tctcaaataa tgctggcctt ttttaaaaag cccttgttct 7440
ttatcaccct gttttctaca taatttttgt tcaaagaaat acttgtttgg atctcctttt 7500
gacaacaata gcatgttttc aagccatatt tttttccctt tttttttttt tttttggttt 7560
ttcgagacag ggtttctctg tatagccctg gctgtcctgg aactcacttt gtagaccagg 7620
ctggcctcga actcagaaat ccgcctgcct ctgcctcctg agtgccggga ttaaaggcgt 7680
gcaccaccac gcctggctaa gttggatatt ttgttatata actataacca atactaactc 7740
cactgggtgg atttttaatt cagtcagtag tcttaagtgg tctttattgg cccttcatta 7800
aaatctactg ttcactctaa cagaggctgt tggtactagt ggcacttaag caacttccta 7860
cggatatact agcagattaa gggtcaggga tagaaactag tctagcgttt tgtataccta 7920

ccagctttat actaccttgt tctgatagaa atatttcagg acatctagag tgtactataa 7980
ggttgatggt aagcttataa ggaacttgaa agtggagtaa ctactccatt tctctgaggg 8040
gagaattaaa atttttgacc aagtgttgtt gagccactga gaatggtctc agaacataac 8100
ttcttaagga accttcccag attgccctca acactgcacc acatttggtc ctgcttgaac 8160
attgccatgg ctcttaaagt cttaattaag aatattaatt gtgtaattat tgtttttcct 8220
cctttagatc attccttgag gacaggacag tgcttgttta aggctatatt tctgctgtct 8280
gagcagcaac aggtcttcga gatcaacatg atgttcataa tcccaagatg ttgccattta 8340
tgttctcaga agcaagcaga ggcatgatgg tcagtgacag taatgtcact gtgttaaatg 8400
ttgctatgca gtttggattt ttctaatgta gtgtaggtag aacatatgtg ttctgtatga 8460
attaaactct taagttacac cttgtataat ccatgcaatg tgttatgcaa ttaccatttt 8520
aagtattgta gctttctttg tatgtgagga taaaggtgtt tgtcataaaa tgttttgaac 8580
atttccccaa agttccaaat tataaaacca caacgttaga acttatttat gaacaatggt 8640
tgtagtttca tgcttttaaa atgcttaatt attcaattaa caccgtttgt gttataatat 8700
atataaaact gacatgtaga agtgtttgtc cagaacattt cttaaatgta tactgtcttt 8760
agagagttta atatagcatg tcttttgcaa catactaact tttgtgttgg tgcgagcaat 8820
attgtgtagt cattttgaaa ggagtcattt caatgagtgt cagattgttt tgaatgttat 8880
tgaacatttt aaatgcagac ttgttcgtgt tttagaaagc aaaactgtca gaagctttga 8940
actagaaatt aaaaagctga agtatttcag aagggaaata agctacttgc tgtattagtt 9000
gaaggaaagt gtaatagctt agaaaattta aaaccatata gttgtcattg ctgaatatct 9060
ggcagatgaa aagaaatact cagtggttct tttgagcaat ataacagctt gttatattaa 9120
aaattttccc cacagatata aactctaatc tataactcat aaatgttaca aatggatgaa 9180
gcttacaaat gtggcttgac ttgtcactgt gcttgtttta gttatgtgaa agtttggcaa 9240
taaacctatg tcctaaatag tcaaactgtg gaatgacttt ttaatctatt ggtttgtcta 9300
gaacagttat gttgccattt gccctaatgg tgaaagaaaa agtggggagt gccttggcac 9360
tgttcatttg tggtgtgaac caaagagggg ggcatgcact tacacttcaa acatcctttt 9420
gaaagactga caagtttggg tcttcacagt tggaattggg catccctttt gtcagggagg 9480
gagggaggga gggaggctgg cttgttatgc tgacaagtgt gattaaattc aaactttgag 9540
gtaagttgga ggaacttgta cattgttagg agtgtgacaa tttggactct taatgatttg 9600
gtcatacaaa atgaacctag accaacttct ggaagatgta tataataact ccatgttaca 9660
ttgatttcac ctgactaata cttatccctt atcaattaaa tacagaagat gccagccatc 9720
tgggcctttt aacccagaaa tttagtttca aactcctagg ttagtgttct cactgagcta 9780
catcctgatc tagtcctgaa aataggacca ccatcacccc caaaaaaatc tcaaataaga 9840
tttatgctag tgtttcaaaa ttttaggaat aggtaagatt agaaagtttt aaattttgag 9900
aaatggcttc tctagaaaga tgtacatagt gaacactgaa tggctcctaa agagcctaga 9960
aaactggtac tgagcacaca ggactgagag gtctttcttg aaaagcatgt attgctttac 10020
gtgggtcaca gaaggcaggc aggaagaact tgggctgaaa ctggtgtctt aagtggctaa 10080
catcttcaca actgatgagc aagaacttta tcctgatgca aaaaccatcc aaacaaacta 10140
agtgaaaggt ggcaatggat cccaggctgc tctagaggag gacttgactt ctcatcccat 10200
cacccacacc agatagctca tagactgcca attaacacca gcttctagcc tccacaggca 10260
cctgcactgg tacacataat ttcacacaaa cacagtaaga agccttccac ctggcatggt 10320
attgcttatc tttagttccc aacacttggg aggcagaggc cagccagggc tatgtgacaa 10380
aaaccttgtc tagaggagaa acttcatagc ttatttccta ttcacgtaac caggttagca 10440
aaatttacca gccagagatg aagctaacag tgtccactat atttgtagtg ttttaagtca 10500
attttttaaa tatacttaat agaattaaag ctatggtgaa ccaagtacaa acctggtgta 10560
ttaacttgag aacttagcat aaaaagtagt tcatttgttc agtaaatatt aaatgcttac 10620
tggcaaagat tatgtcagga acttggtaaa tggtgatgaa acaatcatag ttgtacatct 10680
tggttctgtg atcaccttgg tttgaggtaa aagtggttcc tttgatcaag gatggaattt 10740
taagtttata ttcaatcaat aatgtattat tttgtgattg caaaattgcc tatctagggt 10800
ataaaacctt taaaaatttc ataataccag ttcattctcc agttactaat tccaaaaagc 10860
cactgactat ggtgccaatg tggattctgt tctcaaagga aggattgtct gtgcccttta 10920
ttctaataga aacatcacac tgaaaatcta agctgaaaga agccagactt tcctaaataa 10980
ataactttcc ataaagctca aacaaggatt acttttagga ggcactgtta aggaactgat 11040
aagtaatgag gttacttata taatgatagt cccacaagac tatctgagga aaaatcagta 11100
caactcgaaa acagaacaac cagctaggca ggaataacag ggctcccaag tcaggaggtc 11160
tatccaacac ccttttctgt tgagggcccc agacctacat attgtataca aacaggaggg 11220
tgggtgattt taactctcct gaggtacctt ggtaaatctt tgtcctgagt aagcagtaca 11280
gtgtacagtt tacattttca tttaaagata cattagctcc ctctaccccc taagactgac 11340
aggcactttg ggggtgggga gggctttgga aaataacgct tccatacact aaaagagaaa 11400
tttctttaat taggcttgtt ggttccatac atctactggt gtttctacta cttagtaata 11460
ttataatagt cacacaagca tctttgctct gtttaggttg tatatttatt ttaaggcaga 11520
tgataaaact gtagatctta aggatgcttt ctgcttctga gatgatacaa agaatttaga 11580

ccataaaaca gtaggttgca caagcaatag aatatggcct aaagtgttct gacacttaga 11640
agccaagcag tgtaggcttc ttaagaaata ccattacaat caccttgcta gaaatcaagc 11700
attctggagt ggtcaagcag tgtaacctgt actgtaagtt acttttctgc tatttttctc 11760
ccaaagcaag ttctttatgc tgatatttcc agtgttagga actacaaata ttaataagtt 11820
gtcttcactc ttttctttac caaggagggt ctcttccttc atcttgatct gaaggatgaa 11880
caaaggcttg agcagtgcgc tttagaagat aaactgcagc atgaaggccc ccgatgttca 11940
cccagactac atggaccttt cgccacacat gtcccattcc agataaggcc tggcacacac 12000
aaaaaacata agtcattagg ctaccagtct gattctaaaa caacctaaaa tcttcccact 12060
taaatgctat gggtggtggg ttggaaagtt gactcagaaa atcacttgct gtttttagag 12120
aggatctggg ttcagtttct gatacattgt ggcttacaac tataactcca gttctagggg 12180
gtccatccaa catcctcttc tgttgagggc accaaataaa tgtattgtgt acaaacaggg 12240
aggtgagtga tttaactctc gtgtatagta ccttggtaaa acatttcttg tcctgagtaa 12300
gcagtacagc tctgcctgtc cctggtctac agacacggct catttcccga aggcaagctg 12360
gatagagatt ccaatttctc ttcttggatc ccatcctata aaagaaggtc aagtttaatc 12420
tattgcaaaa ggtaaatagg tagtttctta catgagacaa gaacaaatct taggtgtgaa 12480
gcagtcatct tttacaggcc agagcctcta ttctatgcca atgaaggaaa ctgttagtcc 12540
agtgttatag agttagtcca gtgtatagtt ttctatcaga acactttttt tttaaacaac 12600
tgcaacttag cttattgaag acaaaccacg agtagaaatc tgtccaagaa gcaagtgctt 12660
ctcagcctac aatgtggaat aggaccatgt aatggtacag tgagtgaaat gaattatggc 12720
atgtttttct gactgagaag acagtacaat aaaaggtaaa ctcatggtat ttatttaaaa 12780
agaatccaat ttctaccttt ttccaaatgg catatctgtt acaataatat ccacagaagc 12840
agttctcagt gggaggttgc agatatccca ctgaacagca tcaatgggca aaccccaggt 12900
tgtttttctg tggagacaaa ggtaagatat ttcaatatat tttcccaagc taatgagatg 12960
gctcagcaaa taatggtact ggccattaag tctcatgacc tgagcttgat cctcagggac 13020
catgtggtac aaggagagac ctaaatcctt cagttggact tcaatcttct accctcatgt 13080
ccacacacaa ataaatacaa taaaaaacat tctgcagtcg aatttctaaa agggcgaat 13139

<210> 4
<211> 1073
<212> DNA
<213> Mus musculus

<220>
<223> Description: 5' arm of Rosa 26

<400> 4
caggccctcc gagcgtggtg gagccgttct gtgagacagc cgggtacgag tcgtgacgct 60
ggaaggggca agcgggtggt gggcaggaat gcggtccgcc ctgcagcaac cggaggggga 120
gggagaaggg agcggaaaag tctccaccgg acgcggccat ggctcggggg ggggggggca 180
gcggaggagc gcttccggcc gacgtctcgt cgctgattgg cttcttttcc tcccgccgtg 240
tgtgaaaaca caaatggcgt gttttggttg gcgtaaggcg cctgtcagtt aacggcagcc 300
ggagtgcgca gccgccggca gcctcgctct gcccactggg tggggcggga ggtaggtggg 360
gtgaggcgag ctggacgtgc gggcgcggtc ggcctctggc ggggcggggg aggggaggga 420
gggtcagcga aagtagctcg cgcgcgagcg gccgcccacc ctccccttcc tctgggggag 480
tcgttttacc cgccgccggc cgggcctcgt cgtctgattg gctctcgggg cccagaaaac 540
tggcccttgc cattggctcg tgttcgtgca agttgagtcc atccgccggc cagcgggggc 600
ggcgaggagg cgctcccagg ttccggccct cccctcggcc ccgcgccgca gagtctggcc 660
gcgcgcccct gcgcaacgtg gcaggaagcg cgcgctgggg gcggggacgg gcagtagggc 720
tgagcggctg cggggcgggt gcaagcacgt ttccgacttg agttgcctca agaggggcgt 780
gctgagccag acctccatcg cgcactccgg ggagtggagg gaaggagcga gggctcagtt 840
gggctgtttt ggaggcagga agcacttgct ctcccaaagt cgctctgagt tgttatcagt 900
aagggagctg cagtggagta ggcggggaga aggccgcacc cttctccgga gggggagggg 960
gagtgttgca atacctttct gggagttctc tgctgcctcc tggcttctga ggaccgccct 1020
gggcctggga gaatcccttc cccctcttcc ctcgtgatct gcaactccag tct 1073

<210> 5
<211> 4333
<212> DNA
<213> Mus musculus

<220>
<223> Description: 3' arm of Rosa 26

<400> 5
   tagaagatgg gcgggagtct tctgggcagg cttaaaggct aacctggtgt gtgggcgttg 60
   tcctgcaggg gaattgaaca ggtgtaaaat tggagggaca agacttccca cagattttcg 120
   gttttgtcgg gaagtttttt aataggggca aataaggaaa atgggaggat aggtagtcat 180
   ctggggtttt atgcagcaaa actacaggtt attattgctt gtgatccgcc tcggagtatt 240
   ttccatcgag gtagattaaa gacatgctca cccgagtttt atactctcct gcttgagatc 300
   cttactacag tatgaaatta cagtgtcgcg agttagacta tgtaagcaga attttaatca 360
   tttttaaaga gcccagtact tcatatccat ttctcccgct ccttctgcag ccttatcaaa 420
   aggtatttta gaacactcat tttagcccca ttttcattta ttatactggc ttatccaacc 480
   cctagacaga gcattggcat tttccctttc ctgatcttag aagtctgatg actcatgaaa 540
   ccagacagat tagttacata caccacaaat cgaggctgta gctggggcct caacactgca 600
   gttcttttat aactccttag tacacttttt gttgatcctt tgccttgatc cttaattttc 660
   agtgtctatc acctctcccg tcagtggtgt tccacatttg ggcctattct cagtccaggg 720
   agttttacaa caatagatgt attgagaatc caacctaaag cttaactttc cactcccatg 780
   aatgcctctc tcctttttct ccatttataa actgagctat taaccattaa tggttccagg 840
   tggatgtctc ctccccatat tacctgatgt atcttacata ttgccaggct gatattttaa 900
   gacattaaaa ggtatatttc attattgagc cacatggtat tgattactgc ttactaaaat 960
   tttgtcattg tacacatctg taaaaggtgg ttccttttgg aatgcaaagt tcaggtgttt 1020
   gttgtctttc ctgacctaag gtcttgtgag cttgtatttt ttctatttaa gcagtgcttt 1080
   ctcttggact ggcttgactc atggcattct acacgttatt gctggtctaa atgtgatttt 1140
   gccaagcttc ttcaggacct ataattttgc ttgacttgta gccaaacaca agtaaaatga 1200
   ttaagcaaca aatgtatttg tgaagcttgg tttttaggtt gttgtgttgt gtgtgcttgt 1260
   gctctataat aatactatcc aggggctgga gaggtggctc ggagttcaag agcacagact 1320
   gctcttccag aagtcctgag ttcaattccc agcaaccaca tggtggctca caaccatctg 1380
   taatgggatc tgatgccctc ttctggtgtg tctgaagacc acaagtgtat tcacattaaa 1440
   taaataaatc ctccttcttc ttcttttttt tttttttaaa gagaatactg tctccagtag 1500
   aatttactga agtaatgaaa tactttgtgt ttgttccaat atggtagcca ataatcaaat 1560
   tactctttaa gcactggaaa tgttaccaag gaactaattt ttatttgaag tgtaactgtg 1620
   gacagaggag ccataactgc agacttgtgg gatacagaag accaatgcag actttaatgt 1680
   cttttctctt acactaagca ataaagaaat aaaaattgaa cttctagtat cctatttgtt 1740
   taaactgcta gctttactta acttttgtgc ttcatctata caaagctgaa agctaagtct 1800
   gcagccatta ctaaacatga aagcaagtaa tgataatttt ggatttcaaa aatgtagggc 1860
   cagagtttag ccagccagtg gtggtgcttg cctttatgcc tttaatccca gcactctgga 1920
   ggcagagaca ggcagatctc tgagtttgag cccagcctgg tctacacatc aagttctatc 1980
   taggatagcc aggaatacac acagaaaccc tgttggggag gggggctctg agatttcata 2040
   aaattataat tgaagcattc cctaatgagc cactatggat gtggctaaat ccgtctacct 2100
   ttctgatgag atttgggtat tattttttct gtctctgctg ttggttgggt cttttgacac 2160
   tgtgggcttt ctttaaagcc tccttcctgc catgtggtct cttgtttgct actaacttcc 2220
   catggcttaa atggcatggc tttttgcctt ctaagggcag ctgctgagat ttgcagcctg 2280
   atttccaggg tggggttggg aaatctttca aacactaaaa ttgtccttta attttttttt 2340
   taaaaaatgg gttatataat aaacctcata aaatagttat gaggagtgag gtggactaat 2400
   attaaatgag tccctcccct ataaaagagc tattaaggct ttttgtctta tacttaactt 2460
   tttttttaaa tgtggtatct ttagaaccaa gggtcttaga gttttagtat acagaaactg 2520
   ttgcatcgct taatcagatt ttctagtttc aaatccagag aatccaaatt cttcacagcc 2580
   aaagtcaaat taagaatttc tgacttttaa tgttaatttg cttactgtga atataaaaat 2640
   gatagctttt cctgaggcag ggtctcacta tgtatctctg cctgatctgc aacaagatat 2700
   gtagactaaa gttctgcctg cttttgtctc ctgaatacta aggttaaaat gtagtaatac 2760
   ttttggaact tgcaggtcag attcttttat aggggacaca ctaagggagc ttgggtgata 2820
   gttggtaaaa tgtgtttcaa gtgatgaaaa cttgaattat tatcaccgca acctactttt 2880
   taaaaaaaaa agccaggcct gttagagcat gcttaaggga tccctaggac ttgctgagca 2940
   cacaagagta gttacttggc aggctcctgg tgagagcata tttcaaaaaa caaggcagac 3000
   aaccaagaaa ctacagttaa ggttacctgt ctttaaacca tctgcatata cacagggata 3060
   ttaaaatatt ccaaataata tttcattcaa gttttccccc atcaaattgg gacatggatt 3120
   tctccggtga ataggcagag ttggaaacta aacaaatgtt ggttttgtga tttgtgaaat 3180
   tgttttcaag tgatagttaa agcccatgag atacagaaca aagctgctat ttcgaggtct 3240
   cttggtttat actcagaagc acttctttgg gtttccctgc actatcctga tcatgtgcta 3300
   ggcctacctt aggctgattg ttgttcaaat aaacttaagt ttcctgtcag gtgatgtcat 3360
   atgatttcat atatcaaggc aaaacatgtt atatatgtta aacatttgta cttaatgtga 3420
   aagttaggtc tttgtgggtt tgatttttaa ttttcaaaac ctgagctaaa taagtcattt 3480
   ttacatgtct tacatttggt ggaattgtat aattgtggtt tgcaggcaag actctctgac 3540
   ctagtaaccc tacctataga gcactttgct gggtcacaag tctaggagtc aagcatttca 3600
   ccttgaagtt gagacgtttt gttagtgtat actagtttat atgttggagg acatgtttat 3660
   ccagaagata ttcaggacta tttttgactg ggctaaggaa ttgattctga ttagcactgt 3720
   tagtgagcat tgagtggcct ttaggcttga attggagtca cttgtatatc tcaaataatg 3780
   ctggcctttt ttaaaaagcc cttgttcttt atcaccctgt tttctacata atttttgttc 3840
   aaagaaatac ttgtttggat ctccttttga caacaatagc atgttttcaa gccatatttt 3900
   ttttcctttt tttttttttt tttggttttt cgagacaggg tttctctgta tagccctggc 3960
   tgtcctggaa ctcactttgt agaccaggct ggcctcgaac tcagaaatcc gcctgcctct 4020
   gcctcctgag tgccgggatt aaaggcgtgc accaccacgc ctggctaagt tggatatttt 4080
   gttatataac tataaccaat actaactcca ctgggtggat ttttaattca gtcagtagtc 4140
   ttaagtggtc tttattggcc cttcattaaa atctactgtt cactctaaca gaggctgttg 4200
   gtactagtgg cacttaagca acttcctacg gatatactag cagattaagg gtcagggata 4260
   gaaactagtc tagcgttttg tatacctacc agctttatac taccttgttc tgatagaaat 4320
   atttcaggac atc 4333

<210> 6
<211> 13544
<212> DNA
<213> Artificial Sequence

<220>
<223> Description of Artificial Sequence: Rosa 26
      targeting vector pOMP3 with zsgreen

<400> 6
   ctagggataa cagggtaata tagccgcggc aggccctccg agcgtggtgg agccgttctg 60
   tgagacagcc gggtacgagt cgtgacgctg gaaggggcaa gcgggtggtg ggcaggaatg 120
   cggtccgccc tgcagcaacc ggagggggag ggagaaggga gcggaaaagt ctccaccgga 180
   cgcggccatg gctcgggggg gggggggcag cggaggagcg cttccggccg acgtctcgtc 240
   gctgattggc ttcttttcct cccgccgtgt gtgaaaacac aaatggcgtg ttttggttgg 300
   cgtaaggcgc ctgtcagtta acggcagccg gagtgcgcag ccgccggcag cctcgctctg 360
   cccactgggt ggggcgggag gtaggtgggg tgaggcgagc tggacgtgcg ggcgcggtcg 420
   gcctctggcg ggcgggggga ggggagggag ggtcagcgaa agtagctcgc gcgcgagcgg 480
   ccgcccaccc tccccttcct ctgggggagt cgttttaccc gccgccggcc gggcctcgtc 540
   gtctgattgg ctctcggggc ccagaaaact ggcccttgcc attggctcgt gttcgtgcaa 600
   gttgagtcca tccgccggcc agcgggggcg gcgaggaggc gctcccaggt tccggccctc 660
   ccctcggccc cgcgccgcag agtctggccg cgcgcccctg cgcaacgtgg caggaagcgc 720
   gcgctggggg cggggacggg cagtagggct gagcggctgc ggggcgggtg caagcacgtt 780
   tccgacttga gttgcctcaa gaggggcgtg ctgagccaga cctccatcgc gcactccggg 840
   gagtggaggg aaggagcgag ggctcagttg ggctgttttg gaggcaggaa gcacttgctc 900
   tcccaaagtc gctctgagtt gttatcagta agggagctgc agtggagtag gcggggagaa 960
   ggccgcaccc ttctccggag gggggagggg agtgttgcaa tacctttctg ggagttctct 1020
   gctgcctcct ggcttctgag gaccgccctg ggcctgggag aatcccttcc ccctcttccc 1080
   tcgtgatctg caactccagt ctttctaggt aaccgatatc cctgcagggg tgacctgcac 1140
   gtctagggcg cagtagtcca gggtttcctt gatgatgtca tacttatcct gtcccttttt 1200
   tttccacagc tcgcggttga ggacaaactc ttcgcggtct ttccagtact cctgcaggtg 1260
   actgactgag tcgactaggg gtaaccataa cttcgtataa tgtatgctat acgaagttat 1320
   ttataatcta gaactagtgg atccacgatt cgagggcccc tgcaggtcaa ttctaccggg 1380
   taggggaggc gcttttccca aggcagtctg gagcatgcgc tttagcagcc ccgctgggca 1440
   cttggcgcta cacaagtggc ctctggcctc gcacacattc cacatccacc ggtaggcgcc 1500
   aaccggctcc gttctttggt ggccccttcg cgccaccttc tactcctccc ctagtcagga 1560
   agttcccccc cgccccgcag ctcgcgtcgt gcaggacgtg acaaatggaa gtagcacgtc 1620
   tcactagtct cgtgcagatg gacagcaccg ctgagcaatg gaagcgggta ggcctttggg 1680
   gcagcggcca atagcagctt tgctccttcg ctttctgggc tcagaggctg ggaaggggtg 1740
   ggtccggggg cgggctcagg gcgggctcag gggcggggcg ggcgcccgaa ggtcctccgg 1800
   aggcccggca ttctgcacgc ttcaaaagcg cacgtctgcc gcgctgttct cctcttcctc 1860
   atctccgggc ctttcgacct gcagccaata tgggatcggc cattgaacaa gatggattgc 1920
   acgcaggttc tccggccgct tgggtggaga ggctattcgg ctatgactgg gcacaacaga 1980
   caatcggctg ctctgatgcc gccgtgttcc ggctgtcagc gcaggggcgc ccggttcttt 2040
   ttgtcaagac cgacctgtcc ggtgccctga atgaactgca ggacgaggca gcgcggctat 2100
   cgtggctggc cacgacgggc gttccttgcg cagctgtgct cgacgttgtc actgaagcgg 2160
   gaagggactg gctgctattg ggcgaagtgc cggggcagga tctcctgtca tctcaccttg 2220
   ctcctgccga gaaagtatcc atcatggctg atgcaatgcg gcggctgcat acgcttgatc 2280
   cggctacctg cccattcgac caccaagcga aacatcgcat cgagcgagca cgtactcgga 2340
   tggaagccgg tcttgtcgat caggatgatc tggacgaaga gcatcagggg ctcgcgccag 2400
   ccgaactgtt cgccaggctc aaggcgcgca tgcccgacgg cgaggatctc gtcgtgaccc 2460
   atggcgatgc ctgcttgccg aatatcatgg tggaaaatgg ccgcttttct ggattcatcg 2520
   actgtggccg gctgggtgtg gcggaccgct atcaggacat agcgttggct acccgtgata 2580
   ttgctgaaga gcttggcggc gaatgggctg accgcttcct cgtgctttac ggtatcgccg 2640
   ctcccgattc gcagcgcatc gccttctatc gccttcttga cgagttcttc tgaggggatc 2700
   gatccgctgt aagtctgcag aaattgatga tctattaaac aataaagatg tccactaaaa 2760
   tggaagtttt tcctgtcata ctttgttaag aagggtgaga acagagtacc tacattttga 2820
   atggaaggat tggagctacg ggggtggggg tggggtggga ttagataaat gcctgctctt 2880
   tactgaaggc tctttactat tgctttatga taatgtttca tagttggata tcataattta 2940
   aacaagcaaa accaaattaa gggccagctc attcctccca ctcatgatct atagatctat 3000
   agatctctcg tgggatcatt gtttttctct tgattcccac tttgtggttc taagtactgt 3060
   ggtttccaaa tgtgtcagtt tcatagcctg aagaacgaga tcagcagcct ctgttccaca 3120
   tacacttcat tctcagtatt gttttgccaa gttctaattc catcagaagc tgactctaga 3180
   tcctgcagga attcatatga taacttcgta taatgtatgc tatacgaagt tatggcgcgc 3240
   cggtaaccga agttcctata ctttctagag aataggaact tcggaatagg aacttcaagc 3300
   ttaagcgcta gaagatgggc gggagtcttc tgggcaggct taaaggctaa cctggtgtgt 3360
   gggcgttgtc ctgcagggga attgaacagg tgtaaaattg gagggacaag acttcccaca 3420
   gattttcggt tttgtcggga agttttttaa taggggcaaa taaggaaaat gggaggatag 3480
   gtagtcatct ggggttttat gcagcaaaac tacaggttat tattgcttgt gatccgcctc 3540
   ggagtatttt ccatcgaggt agattaaaga catgctcacc cgagttttat actctcctgc 3600
   ttgagatcct tactacagta tgaaattaca gtgtcgcgag ttagactatg taagcagaat 3660
   tttaatcatt tttaaagagc ccagtacttc atatccattt ctcccgctcc ttctgcagcc 3720
   ttatcaaaag gtattttaga acactcattt tagccccatt ttcatttatt atactggctt 3780
   atccaacccc tagacagagc attggcattt tccctttcct gatcttagaa gtctgatgac 3840
   tcatgaaacc agacagatta gttacataca ccacaaatcg aggctgtagc tggggcctca 3900
   acactgcagt tcttttataa ctccttagta cactttttgt tgatcctttg ccttgatcct 3960
   taattttcag tgtctatcac ctctcccgtc agtggtgttc cacatttggg cctattctca 4020
   gtccagggag ttttacaaca atagatgtat tgagaatcca acctaaagct taactttcca 4080
   ctcccatgaa tgcctctctc ctttttctcc atttataaac tgagctatta accattaatg 4140
   gttccaggtg gatgtctcct ccccatatta cctgatgtat cttacatatt gccaggctga 4200
   tattttaaga cattaaaagg tatatttcat tattgagcca catggtattg attactgctt 4260
   actaaaattt tgtcattgta cacatctgta aaaggtggtt ccttttggaa tgcaaagttc 4320
   aggtgtttgt tgtctttcct gacctaaggt cttgtgagct tgtatttttt ctatttaagc 4380
   agtgctttct cttggactgg cttgactcat ggcattctac acgttattgc tggtctaaat 4440
   gtgattttgc caagcttctt caggacctat aattttgctt gacttgtagc caaacacaag 4500
   taaaatgatt aagcaacaaa tgtatttgtg aagcttggtt tttaggttgt tgtgttgtgt 4560
   gtgcttgtgc tctataataa tactatccag gggctggaga ggtggctcgg agttcaagag 4620
   cacagactgc tcttccagaa gtcctgagtt caattcccag caaccacatg gtggctcaca 4680
   accatctgta atgggatctg atgccctctt ctggtgtgtc tgaagaccac aagtgtattc 4740
   acattaaata aataaatcct ccttcttctt cttttttttt tttttaaaga gaatactgtc 4800
   tccagtagaa tttactgaag taatgaaata ctttgtgttt gttccaatat ggtagccaat 4860
   aatcaaatta ctctttaagc actggaaatg ttaccaagga actaattttt atttgaagtg 4920
   taactgtgga cagaggagcc ataactgcag acttgtggga tacagaagac caatgcagac 4980
   tttaatgtct tttctcttac actaagcaat aaagaaataa aaattgaact tctagtatcc 5040
   tatttgttta aactgctagc tttacttaac ttttgtgctt catctataca aagctgaaag 5100
   ctaagtctgc agccattact aaacatgaaa gcaagtaatg ataattttgg atttcaaaaa 5160
   tgtagggcca gagtttagcc agccagtggt ggtgcttgcc tttatgcctt taatcccagc 5220
   actctggagg cagagacagg cagatctctg agtttgagcc cagcctggtc tacacatcaa 5280
   gttctatcta ggatagccag gaatacacac agaaaccctg ttggggaggg gggctctgag 5340
   atttcataaa attataattg aagcattccc taatgagcca ctatggatgt ggctaaatcc 5400
   gtctaccttt ctgatgagat ttgggtatta ttttttctgt ctctgctgtt ggttgggtct 5460
   tttgacactg tgggctttct ttaaagcctc cttcctgcca tgtggtctct tgtttgctac 5520
   taacttccca tggcttaaat ggcatggctt tttgccttct aagggcagct gctgagattt 5580
   gcagcctgat ttccagggtg gggttgggaa atctttcaaa cactaaaatt gtcctttaat 5640
   ttttttttta aaaaatgggt tatataataa acctcataaa atagttatga ggagtgaggt 5700
   ggactaatat taaatgagtc cctcccctat aaaagagcta ttaaggcttt ttgtcttata 5760
   cttaactttt tttttaaatg tggtatcttt agaaccaagg gtcttagagt tttagtatac 5820
   agaaactgtt gcatcgctta atcagatttt ctagtttcaa atccagagaa tccaaattct 5880
   tcacagccaa agtcaaatta agaatttctg acttttaatg ttaatttgct tactgtgaat 5940
   ataaaaatga tagcttttcc tgaggcaggg tctcactatg tatctctgcc tgatctgcaa 6000
   caagatatgt agactaaagt tctgcctgct tttgtctcct gaatactaag gttaaaatgt 6060
   agtaatactt ttggaacttg caggtcagat tcttttatag gggacacact aagggagctt 6120
   gggtgatagt tggtaaaatg tgtttcaagt gatgaaaact tgaattatta tcaccgcaac 6180
   ctacttttta aaaaaaaaag ccaggcctgt tagagcatgc ttaagggatc cctaggactt 6240
   gctgagcaca caagagtagt tacttggcag gctcctggtg agagcatatt tcaaaaaaca 6300
   aggcagacaa ccaagaaact acagttaagg ttacctgtct ttaaaccatc tgcatataca 6360
   cagggatatt aaaatattcc aaataatatt tcattcaagt tttcccccat caaattggga 6420
   catggatttc tccggtgaat aggcagagtt ggaaactaaa caaatgttgg ttttgtgatt 6480
   tgtgaaattg ttttcaagtg atagttaaag cccatgagat acagaacaaa gctgctattt 6540
   cgaggtctct tggtttatac tcagaagcac ttctttgggt ttccctgcac tatcctgatc 6600
   atgtgctagg cctaccttag gctgattgtt gttcaaataa acttaagttt cctgtcaggt 6660
   gatgtcatat gatttcatat atcaaggcaa aacatgttat atatgttaaa catttgtact 6720
   taatgtgaaa gttaggtctt tgtgggtttg atttttaatt ttcaaaacct gagctaaata 6780
   agtcattttt acatgtctta catttggtgg aattgtataa ttgtggtttg caggcaagac 6840
   tctctgacct agtaacccta cctatagagc actttgctgg gtcacaagtc taggagtcaa 6900
   gcatttcacc ttgaagttga gacgttttgt tagtgtatac tagtttatat gttggaggac 6960
   atgtttatcc agaagatatt caggactatt tttgactggg ctaaggaatt gattctgatt 7020
   agcactgtta gtgagcattg agtggccttt aggcttgaat tggagtcact tgtatatctc 7080
   aaataatgct ggcctttttt aaaaagccct tgttctttat caccctgttt tctacataat 7140
   ttttgttcaa agaaatactt gtttggatct ccttttgaca acaatagcat gttttcaagc 7200
   catatttttt ttcctttttt tttttttttt tggtttttcg agacagggtt tctctgtata 7260
   gccctggctg tcctggaact cactttgtag accaggctgg cctcgaactc agaaatccgc 7320
   ctgcctctgc ctcctgagtg ccgggattaa aggcgtgcac caccacgcct ggctaagttg 7380
   gatattttgt tatataacta taaccaatac taactccact gggtggattt ttaattcagt 7440
   cagtagtctt aagtggtctt tattggccct tcattaaaat ctactgttca ctctaacaga 7500
   ggctgttggt actagtggca cttaagcaac ttcctacgga tatactagca gattaagggt 7560
   cagggataga aactagtcta gcgttttgta tacctaccag ctttatacta ccttgttctg 7620
   atagaaatat ttcaggacat ctagcacggg caccggtgtt tttaagatac attgatgagt 7680
   ttggacaaac cacaactaga atgcagtgaa aaaaatgctt tatttgtgaa atttgtgatg 7740
   ctattgcttt atttgtaacc attataagct gcaataaaca agttaacaac aacaattgca 7800
   ttcattttat gtttcaggtt cagggggagg tgtgggaggt tttttaaagc aagtaaaacc 7860
   tctacaaatg tggtatggct gattatgatc tagagtcgcg gccgctcagg gcaaggcgga 7920
   gccggaggcg atggcgtgct cggtcaggtg ccacttctgg ttcttggcgt cgctgcggtc 7980
   ctcgcgggtc agcttgtgct ggatgaagtg ccagtcgggc atcttgcggg gcacggactt 8040
   ggccttgtac acggtgtcga actggcagcg caagcggcca ccgtccttca gcagcaggta 8100
   catgctcacg tcgcccttca agatgccctg cttgggcacg gggatgatct tctcgcagga 8160
   gggctcccag ttgtcggtca tcttcttcat cacggggccg tcggcgggga agttcacgcc 8220
   gtagaacttg gactcgtggt acatgcagtt ctcctccacg ctcacggtga tgtcggcgtt 8280
   gcagatgcac acggcgccgt cctcgaacag gaaggagcgg tcccaggtgt agccggcggg 8340
   gcaggagttc ttgaagtagt cgacgatgtc ctgggggtac tcggtgaaca cgcggttgcc 8400
   gtacatgaag gcggcggaca agatgtcctc ggcgaagggc aaggggccgc cctccaccac 8460
   gcacaggttg atggcctgct tgcccttgaa ggggtagccg atgccctcgc cggtgatcac 8520
   gaacttgtgg ccgtccacgc agccctccat gcggtacttc atggtcatct ccttggtcag 8580
   gccgtgcttg gactgggcca tggtggcgac cggtggatcc actagttcta gctgtaggaa 8640
   aaagaagaag gcatgaacat ggttagcaga ggctctaggg ccgccctggg agtggacacc 8700
   tgtggagaga aaggcaaagt ggatgtcaat gtcactcaag tgtatggcca gatctcaagc 8760
   ctgccacacc tcaagcttga caacaaaaag attgtctttt ctgaccagat ggacgcggcc 8820
   accctcaaag gcatcaccgc gggccaggtg aatatcaaat cctcctcgtt tttggaaact 8880
   gacaatctta gcgcagaagt catgcccgct tttgagaggg agtactcacc ccaacgctta 8940
   ctagagccgc cggtcacacg ccagaagccg aaccccgccc tgccccgtcc cccccgaagg 9000
   cagccgtccc cctgcggcag ccccgaggct ggagatggag aaggggacgg cggcgcggcg 9060
   acgcacgaag gccctccccg cccatttcct tcctgccggc gccgcaccgc ttcgcccgcg 9120
   cccgctagag ggggtgcggc ggcgcctccc agatttcggc tccgccagat ttgggacaaa 9180
   ggaagtccct gcgccctctc gcacgattac cataaaaggc aatggctgcg gctcgccgcg 9240
   cctcgacagc cgccggcgct ccggggccgc cgcgcccctc ccccgagccc tccccggccc 9300
   gaggcggccc cgccccgccc ggcaccccca cctgccgcca ccccccgccc ggcacggcga 9360
   gccccgcgcc acgccccgca cggagccccg cacccgaagc cgggccgtgc tcagcaactc 9420
   ggggaggggg gtgcaggggg gggttacagc ccgaccgccg cgcccacacc ccctgctcac 9480
   ccccccacgc acacaccccg cacgcagcct ttgttcccct cgcagccccc ccgcaccgcg 9540
   gggcaccgcc cccggccgcg ctcccctcgc gcacacgcgg agcgcacaaa gccccgcgcc 9600
   gcgcccgcag cgctcacagc cgccgggcag cgcgggccgc acgcggcgct ccccacgcac 9660
   acacacacgc acgcaccccc cgagccgctc ccccccgcac aaagggccct cccggagccc 9720
   tttaaggctt tcacgcagcc acagaaaaga aacgagccgt cattaaacca agcgctaatt 9780
   acagcccgga ggagaagggc cgtcccgccc gctcacctgt gggagtaacg cggtcagtca 9840
   gagccggggc gggcggcgcg aggcggcgcg gagcggggca cggggcgaag gcaacgcagc 9900
   gactcccgcc cgccgcgcgc ttcgcttttt atagggccgc cgccgccgcc gcctcgccat 9960
   aaaaggaaac tttcggagcg cgccgctctg attggctgcc gccgcacctc tccgcctcgc 10020
   cccgccccgc ccctcgcccc gccccgcccc gcctggcgcg cgcccccccc cccccgcccc 10080
   ccatcgctgc acaaaataat taaaaaataa ataaatacaa aattgggggt ggggaggggg 10140
   gggagatggg gagagtgaag cagaacgtgg ggctcacctc gacccatggt aatagcgatg 10200
   actaatacgt agatgtactg ccaagtagga aagtcccata aggtcatgta ctgggcataa 10260
   tgccaggcgg gccatttacc gtcattgacg tcaatagggg gcgtacttgg catatgatac 10320
   acttgatgta ctgccaagtg ggcagtttac cgtaaatagt ccacccattg acgtcaatgg 10380
   aaagtcccta ttggcgttac tatgggaaca tacgtcatta ttgacgtcaa tgggcggggg 10440
   tcgttgggcg gtcagccagg cgggccattt accgtaagtt atgtaacgcg gaactccata 10500
   tatgggctat gaactaatga ccccgtaatt gattactatt aataactagt caataatcaa 10560
   tgtcgaaaac ctgcaggcat gcaagctagc ccaaacctat attaccctgc cctagcgtaa 10620
   ctatttaaat ggccggcccc tgcaggccgc ggtggagctc caattcgccc tatagtgagt 10680
   cgtattacaa ttcactggcc gtcgttttac aacgtcgtga ctgggaaaac cctggcgtta 10740
   cccaacttaa tcgccttgca gcacatcccc ctttcgccag ctggcgtaat agcgaagagg 10800
   cccgcaccga tcgcccttcc caacagttgc gcagcctgaa tggcgaatgg gacgcgccct 10860
   gtagcggcgc attaagcgcg gcgggtgtgg tggttacgcg cagcgtgacc gctacacttg 10920
   ccagcgccct agcgcccgct cctttcgctt tcttcccttc ctttctcgcc acgttcgccg 10980
   gctttccccg tcaagctcta aatcgggggc tccctttagg gttccgattt agtgctttac 11040
   ggcacctcga ccccaaaaaa cttgattagg gtgatggttc acgtagtggg ccatcgccct 11100
   gatagacggt ttttcgccct ttgacgttgg agtccacgtt ctttaatagt ggactcttgt 11160
   tccaaactgg aacaacactc aaccctatct cggtctattc ttttgattta taagggattt 11220
   tgccgatttc ggcctattgg ttaaaaaatg agctgattta acaaaaattt aacgcgaatt 11280
   ttaacaaaat attaacgctt acaatttagg tggcactttt cggggaaatg tgcgcggaac 11340
   ccctatttgt ttatttttct aaatacattc aaatatgtat ccgctcatga gacaataacc 11400
   ctgataaatg cttcaataat attgaaaaag gaagagtatg agtattcaac atttccgtgt 11460
   cgcccttatt cccttttttg cggcattttg ccttcctgtt tttgctcacc cagaaacgct 11520
   ggtgaaagta aaagatgctg aagatcagtt gggtgcacga gtgggttaca tcgaactgga 11580
   tctcaacagc ggtaagatcc ttgagagttt tcgccccgaa gaacgttttc caatgatgag 11640
   cacttttaaa gttctgctat gtggcgcggt attatcccgt attgacgccg ggcaagagca 11700
   actcggtcgc cgcatacact attctcagaa tgacttggtt gagtactcac cagtcacaga 11760
   aaagcatctt acggatggca tgacagtaag agaattatgc agtgctgcca taaccatgag 11820
   tgataacact gcggccaact tacttctgac aacgatcgga ggaccgaagg agctaaccgc 11880
   ttttttgcac aacatggggg atcatgtaac tcgccttgat cgttgggaac cggagctgaa 11940
   tgaagccata ccaaacgacg agcgtgacac cacgatgcct gtagcaatgg caacaacgtt 12000
   gcgcaaacta ttaactggcg aactacttac tctagcttcc cggcaacaat taatagactg 12060
   gatggaggcg gataaagttg caggaccact tctgcgctcg gcccttccgg ctggctggtt 12120
   tattgctgat aaatctggag ccggtgagcg tgggtctcgc ggtatcattg cagcactggg 12180
   gccagatggt aagccctccc gtatcgtagt tatctacacg acggggagtc aggcaactat 12240
   ggatgaacga aatagacaga tcgctgagat aggtgcctca ctgattaagc attggtaact 12300
   gtcagaccaa gtttactcat atatacttta gattgattta aaacttcatt tttaatttaa 12360
   aaggatctag gtgaagatcc tttttgataa tctcatgacc aaaatccctt aacgtgagtt 12420
   ttcgttccac tgagcgtcag accccgtaga aaagatcaaa ggatcttctt gagatccttt 12480
   ttttctgcgc gtaatctgct gcttgcaaac aaaaaaacca ccgctaccag cggtggtttg 12540
   tttgccggat caagagctac caactctttt tccgaaggta actggcttca gcagagcgca 12600
   gataccaaat actgtccttc tagtgtagcc gtagttaggc caccacttca agaactctgt 12660
   agcaccgcct acatacctcg ctctgctaat cctgttacca gtggctgctg ccagtggcga 12720
   taagtcgtgt cttaccgggt tggactcaag acgatagtta ccggataagg cgcagcggtc 12780
   gggctgaacg gggggttcgt gcacacagcc cagcttggag cgaacgacct acaccgaact 12840
   gagataccta cagcgtgagc tatgagaaag cgccacgctt cccgaaggga gaaaggcgga 12900
   caggtatccg gtaagcggca gggtcggaac aggagagcgc acgagggagc ttccaggggg 12960
   aaacgcctgg tatctttata gtcctgtcgg gtttcgccac ctctgacttg agcgtcgatt 13020
   tttgtgatgc tcgtcagggg ggcggagcct atggaaaaac gccagcaacg cggccttttt 13080
   acggttcctg gccttttgct ggccttttgc tcacatgttc tttcctgcgt tatcccctga 13140
   ttctgtggat aaccgtatta ccgcctttga gtgagctgat accgctcgcc gcagccgaac 13200
   gaccgagcgc agcgagtcag tgagcgagga agcggaagag cgcccaatac gcaaaccgcc 13260
   tctccccgcg cgttggccga ttcattaatg cagctggcac gacaggtttc ccgactggaa 13320
   agcgggcagt gagcgcaacg caattaatgt gagttagctc actcattagg caccccaggc 13380
   tttacacttt atgcttccgg ctcgtatgtt gtgtggaatt gtgagcggat aacaatttca 13440
   cacaggaaac agctatgacc atgattacgc caagcgcgca attaaccctc actaaaggga 13500
   acaaaagctg tcgagatcta gatatcgatg gccatagagt tacg 13544

<210> 7
<211> 12739
<212> DNA
<213> Artificial Sequence

<220>
<223> Description of Artificial Sequence: Rosa 26
      targeting vector pOMP1 with TK

<400> 7
   ctagggataa cagggtaata tagccgcggc aggccctccg agcgtggtgg agccgttctg 60
   tgagacagcc gggtacgagt cgtgacgctg gaaggggcaa gcgggtggtg ggcaggaatg 120
   cggtccgccc tgcagcaacc ggagggggag ggagaaggga gcggaaaagt ctccaccgga 180
   cgcggccatg gctcgggggg gggggggcag cggaggagcg cttccggccg acgtctcgtc 240
   gctgattggc ttcttttcct cccgccgtgt gtgaaaacac aaatggcgtg ttttggttgg 300
   cgtaaggcgc ctgtcagtta acggcagccg gagtgcgcag ccgccggcag cctcgctctg 360
   cccactgggt ggggcgggag gtaggtgggg tgaggcgagc tggacgtgcg ggcgcggtcg 420
   gcctctggcg gggcggggga ggggagggag ggtcagcgaa agtagctcgc gcgcgagcgg 480
   ccgcccaccc tccccttcct ctgggggagt cgttttaccc gccgccggcc gggcctcgtc 540
   gtctgattgg ctctcggggc ccagaaaact ggcccttgcc attggctcgt gttcgtgcaa 600
   gttgagtcca tccgccggcc agcgggggcg gcgaggaggc gctcccaggt tccggccctc 660
   ccctcggccc cgcgccgcag agtctggccg cgcgcccctg cgcaacgtgg caggaagcgc 720
   gcgctggggg cggggacggg cagtagggct gagcggctgc ggggcgggtg caagcacgtt 780
   tccgacttga gttgcctcaa gaggggcgtg ctgagccaga cctccatcgc gcactccggg 840
   gagtggaggg aaggagcgag ggctcagttg ggctgttttg gaggcaggaa gcacttgctc 900
   tcccaaagtc gctctgagtt gttatcagta agggagctgc agtggagtag gcggggagaa 960
   ggccgcaccc ttctccggag gggggagggg agtgttgcaa tacctttctg ggagttctct 1020
   gctgcctcct ggcttctgag gaccgccctg ggcctgggag aatcccttcc ccctcttccc 1080
   tcgtgatctg caactccagt ctttctaggt aaccgatatc cctgcagggg tgacctgcac 1140
   gtctagggcg cagtagtcca gggtttcctt gatgatgtca tacttatcct gtcccttttt 1200
   tttccacagc tcgcggttga ggacaaactc ttcgcggtct ttccagtact cctgcaggtg 1260
   actgactgag tcgactaggg gtaaccataa cttcgtataa tgtatgctat acgaagttat 1320
   ttataatcta gaactagtgg atccacgatt cgagggcccc tgcaggtcaa ttctaccggg 1380
   taggggaggc gcttttccca aggcagtctg gagcatgcgc tttagcagcc ccgctgggca 1440
   cttggcgcta cacaagtggc ctctggcctc gcacacattc cacatccacc ggtaggcgcc 1500
   aaccggctcc gttctttggt ggccccttcg cgccaccttc tactcctccc ctagtcagga 1560
   agttcccccc cgccccgcag ctcgcgtcgt gcaggacgtg acaaatggaa gtagcacgtc 1620
   tcactagtct cgtgcagatg gacagcaccg ctgagcaatg gaagcgggta ggcctttggg 1680
   gcagcggcca atagcagctt tgctccttcg ctttctgggc tcagaggctg ggaaggggtg 1740
   ggtccggggg cgggctcagg gcgggctcag gggcggggcg ggcgcccgaa ggtcctccgg 1800
   aggcccggca ttctgcacgc ttcaaaagcg cacgtctgcc gcgctgttct cctcttcctc 1860
   atctccgggc ctttcgacct gcagccaata tgggatcggc cattgaacaa gatggattgc 1920
   acgcaggttc tccggccgct tgggtggaga ggctattcgg ctatgactgg gcacaacaga 1980
   caatcggctg ctctgatgcc gccgtgttcc ggctgtcagc gcaggggcgc ccggttcttt 2040
   ttgtcaagac cgacctgtcc ggtgccctga atgaactgca ggacgaggca gcgcggctat 2100
   cgtggctggc cacgacgggc gttccttgcg cagctgtgct cgacgttgtc actgaagcgg 2160
   gaagggactg gctgctattg ggcgaagtgc cggggcagga tctcctgtca tctcaccttg 2220
   ctcctgccga gaaagtatcc atcatggctg atgcaatgcg gcggctgcat acgcttgatc 2280
   cggctacctg cccattcgac caccaagcga aacatcgcat cgagcgagca cgtactcgga 2340
   tggaagccgg tcttgtcgat caggatgatc tggacgaaga gcatcagggg ctcgcgccag 2400
   ccgaactgtt cgccaggctc aaggcgcgca tgcccgacgg cgaggatctc gtcgtgaccc 2460
   atggcgatgc ctgcttgccg aatatcatgg tggaaaatgg ccgcttttct ggattcatcg 2520
   actgtggccg gctgggtgtg gcggaccgct atcaggacat agcgttggct acccgtgata 2580
   ttgctgaaga gcttggcggc gaatgggctg accgcttcct cgtgctttac ggtatcgccg 2640
   ctcccgattc gcagcgcatc gccttctatc gccttcttga cgagttcttc tgaggggatc 2700
   gatccgctgt aagtctgcag aaattgatga tctattaaac aataaagatg tccactaaaa 2760
   tggaagtttt tcctgtcata ctttgttaag aagggtgaga acagagtacc tacattttga 2820
   atggaaggat tggagctacg ggggtggggg tggggtggga ttagataaat gcctgctctt 2880
   tactgaaggc tctttactat tgctttatga taatgtttca tagttggata tcataattta 2940
   aacaagcaaa accaaattaa gggccagctc attcctccca ctcatgatct atagatctat 3000
   agatctctcg tgggatcatt gtttttctct tgattcccac tttgtggttc taagtactgt 3060
   ggtttccaaa tgtgtcagtt tcatagcctg aagaacgaga tcagcagcct ctgttccaca 3120
   tacacttcat tctcagtatt gttttgccaa gttctaattc catcagaagc tgactctaga 3180
   tcctgcagga attcatatga taacttcgta taatgtatgc tatacgaagt tatggcgcgc 3240
   cggtaaccga agttcctata ctttctagag aataggaact tcggaatagg aacttcaagc 3300
   ttaagcgcta gaagatgggc gggagtcttc tgggcaggct taaaggctaa cctggtgtgt 3360
   gggcgttgtc ctgcagggga attgaacagg tgtaaaattg gagggacaag acttcccaca 3420
   gattttcggt tttgtcggga agttttttaa taggggcaaa taaggaaaat gggaggatag 3480
   gtagtcatct ggggttttat gcagcaaaac tacaggttat tattgcttgt gatccgcctc 3540
   ggagtatttt ccatcgaggt agattaaaga catgctcacc cgagttttat actctcctgc 3600
   ttgagatcct tactacagta tgaaattaca gtgtcgcgag ttagactatg taagcagaat 3660
   tttaatcatt tttaaagagc ccagtacttc atatccattt ctcccgctcc ttctgcagcc 3720
   ttatcaaaag gtattttaga acactcattt tagccccatt ttcatttatt atactggctt 3780
   atccaacccc tagacagagc attggcattt tccctttcct gatcttagaa gtctgatgac 3840
   tcatgaaacc agacagatta gttacataca ccacaaatcg aggctgtagc tggggcctca 3900
   acactgcagt tcttttataa ctccttagta cactttttgt tgatcctttg ccttgatcct 3960
   taattttcag tgtctatcac ctctcccgtc agtggtgttc cacatttggg cctattctca 4020
   gtccagggag ttttacaaca atagatgtat tgagaatcca acctaaagct taactttcca 4080
   ctcccatgaa tgcctctctc ctttttctcc atttataaac tgagctatta accattaatg 4140
   gttccaggtg gatgtctcct ccccatatta cctgatgtat cttacatatt gccaggctga 4200
   tattttaaga cattaaaagg tatatttcat tattgagcca catggtattg attactgctt 4260
   actaaaattt tgtcattgta cacatctgta aaaggtggtt ccttttggaa tgcaaagttc 4320
   aggtgtttgt tgtctttcct gacctaaggt cttgtgagct tgtatttttt ctatttaagc 4380
   agtgctttct cttggactgg cttgactcat ggcattctac acgttattgc tggtctaaat 4440
   gtgattttgc caagcttctt caggacctat aattttgctt gacttgtagc caaacacaag 4500
   taaaatgatt aagcaacaaa tgtatttgtg aagcttggtt tttaggttgt tgtgttgtgt 4560
   gtgcttgtgc tctataataa tactatccag gggctggaga ggtggctcgg agttcaagag 4620
   cacagactgc tcttccagaa gtcctgagtt caattcccag caaccacatg gtggctcaca 4680
   accatctgta atgggatctg atgccctctt ctggtgtgtc tgaagaccac aagtgtattc 4740
   acattaaata aataaatcct ccttcttctt cttttttttt tttttaaaga gaatactgtc 4800
   tccagtagaa tttactgaag taatgaaata ctttgtgttt gttccaatat ggtagccaat 4860
   aatcaaatta ctctttaagc actggaaatg ttaccaagga actaattttt atttgaagtg 4920
   taactgtgga cagaggagcc ataactgcag acttgtggga tacagaagac caatgcagac 4980
   tttaatgtct tttctcttac actaagcaat aaagaaataa aaattgaact tctagtatcc 5040
   tatttgttta aactgctagc tttacttaac ttttgtgctt catctataca aagctgaaag 5100
   ctaagtctgc agccattact aaacatgaaa gcaagtaatg ataattttgg atttcaaaaa 5160
   tgtagggcca gagtttagcc agccagtggt ggtgcttgcc tttatgcctt taatcccagc 5220
   actctggagg cagagacagg cagatctctg agtttgagcc cagcctggtc tacacatcaa 5280
   gttctatcta ggatagccag gaatacacac agaaaccctg ttggggaggg gggctctgag 5340
   atttcataaa attataattg aagcattccc taatgagcca ctatggatgt ggctaaatcc 5400
   gtctaccttt ctgatgagat ttgggtatta ttttttctgt ctctgctgtt ggttgggtct 5460
   tttgacactg tgggctttct ttaaagcctc cttcctgcca tgtggtctct tgtttgctac 5520
   taacttccca tggcttaaat ggcatggctt tttgccttct aagggcagct gctgagattt 5580
   gcagcctgat ttccagggtg gggttgggaa atctttcaaa cactaaaatt gtcctttaat 5640
   ttttttttta aaaaatgggt tatataataa acctcataaa atagttatga ggagtgaggt 5700
   ggactaatat taaatgagtc cctcccctat aaaagagcta ttaaggcttt ttgtcttata 5760
   cttaactttt tttttaaatg tggtatcttt agaaccaagg gtcttagagt tttagtatac 5820
   agaaactgtt gcatcgctta atcagatttt ctagtttcaa atccagagaa tccaaattct 5880
   tcacagccaa agtcaaatta agaatttctg acttttaatg ttaatttgct tactgtgaat 5940
   ataaaaatga tagcttttcc tgaggcaggg tctcactatg tatctctgcc tgatctgcaa 6000
   caagatatgt agactaaagt tctgcctgct tttgtctcct gaatactaag gttaaaatgt 6060
   agtaatactt ttggaacttg caggtcagat tcttttatag gggacacact aagggagctt 6120
   gggtgatagt tggtaaaatg tgtttcaagt gatgaaaact tgaattatta tcaccgcaac 6180
   ctacttttta aaaaaaaaag ccaggcctgt tagagcatgc ttaagggatc cctaggactt 6240
   gctgagcaca caagagtagt tacttggcag gctcctggtg agagcatatt tcaaaaaaca 6300
   aggcagacaa ccaagaaact acagttaagg ttacctgtct ttaaaccatc tgcatataca 6360
   cagggatatt aaaatattcc aaataatatt tcattcaagt tttcccccat caaattggga 6420
   catggatttc tccggtgaat aggcagagtt ggaaactaaa caaatgttgg ttttgtgatt 6480
   tgtgaaattg ttttcaagtg atagttaaag cccatgagat acagaacaaa gctgctattt 6540
   cgaggtctct tggtttatac tcagaagcac ttctttgggt ttccctgcac tatcctgatc 6600
   atgtgctagg cctaccttag gctgattgtt gttcaaataa acttaagttt cctgtcaggt 6660
   gatgtcatat gatttcatat atcaaggcaa aacatgttat atatgttaaa catttgtact 6720
   taatgtgaaa gttaggtctt tgtgggtttg atttttaatt ttcaaaacct gagctaaata 6780
   agtcattttt acatgtctta catttggtgg aattgtataa ttgtggtttg caggcaagac 6840
   tctctgacct agtaacccta cctatagagc actttgctgg gtcacaagtc taggagtcaa 6900
   gcatttcacc ttgaagttga gacgttttgt tagtgtatac tagtttatat gttggaggac 6960
   atgtttatcc agaagatatt caggactatt tttgactggg ctaaggaatt gattctgatt 7020
   agcactgtta gtgagcattg agtggccttt aggcttgaat tggagtcact tgtatatctc 7080
   aaataatgct ggcctttttt aaaaagccct tgttctttat caccctgttt tctacataat 7140
   ttttgttcaa agaaatactt gtttggatct ccttttgaca acaatagcat gttttcaagc 7200
   catatttttt ttcctttttt tttttttttt tggtttttcg agacagggtt tctctgtata 7260
   gccctggctg tcctggaact cactttgtag accaggctgg cctcgaactc agaaatccgc 7320
   ctgcctctgc ctcctgagtg ccgggattaa aggcgtgcac caccacgcct ggctaagttg 7380
   gatattttgt tatataacta taaccaatac taactccact gggtggattt ttaattcagt 7440
   cagtagtctt aagtggtctt tattggccct tcattaaaat ctactgttca ctctaacaga 7500
   ggctgttggt actagtggca cttaagcaac ttcctacgga tatactagca gattaagggt 7560
   cagggataga aactagtcta gcgttttgta tacctaccag ctttatacta ccttgttctg 7620
   atagaaatat ttcaggacat ctagcacgtg ttaactcgag ctgcaggatt cgagggcccc 7680
   ggcaggtcaa ttctaccggg taggggaggc gcttttccca aggcagtctg gagcatgcgc 7740
   tttagcagcc ccgctgggca cttggcgcta cacaagtggc ctctggcctc gcacacattc 7800
   cacatccacc ggtaggcgcc aaccggctcc gttctttggt ggccccttcg cgccaccttc 7860
   tactcctccc ctagtcagga agttcccccc cgccccgcag ctcgcgtcgt gcaggacgtg 7920
   acaaatggaa gtagcacgtc tcactagtct cgtgcagatg gacagcaccg ctgagcaatg 7980
   gaagcgggta ggcctttggg gcagcggcca atagcagctt tgctccttcg ctttctgggc 8040
   tcagaggctg ggaaggggtg ggtccggggg cgggctcagg ggcgggctca ggggcggggc 8100
   gggcgcccga aggtcctccg gaggcccggc attctgcacg cttcaaaagc gcacgtctgc 8160
   cgcgctgttc tcctcttcct catctccggg cctttcgacc tgcagccaat gcaccgtcct 8220
   tgccatcatg gcctcgtacc ccggccatca acacgcgtct gcgttcgacc aggctgcgcg 8280
   ttctcgcggc catagcaacc gacgtacggc gttgcgccct cgccggcagc aagaagccac 8340
   ggaagtccgc ccggagcaga aaatgcccac gctactgcgg gtttatatag acggtcccca 8400
   cgggatgggg aaaaccacca ccacgcaact gctggtggcc ctgggttcgc gcgacgatat 8460
   cgtctacgta cccgagccga tgacttactg gcgggtgctg ggggcttccg agacaatcgc 8520
   gaacatctac accacacaac accgcctcga ccagggtgag atatcggccg gggacgcggc 8580
   ggtggtaatg acaagcgccc agataacaat gggcatgcct tatgccgtga ccgacgccgt 8640
   tctggctcct catatcgggg gggaggctgg gagctcacat gccccgcccc cggccctcac 8700
   cctcatcttc gaccgccatc ccatcgccgc cctcctgtgc tacccggccg cgcggtacct 8760
   tatgggcagc atgacccccc aggccgtgct ggcgttcgtg gccctcatcc cgccgacctt 8820
   gcccggcacc aacatcgtgc ttggggccct tccggaggac agacacatcg accgcctggc 8880
   caaacgccag cgccccggcg agcggctgga cctggctatg ctggctgcga ttcgccgcgt 8940
   ttacgggcta cttgccaata cggtgcggta tctgcagtgc ggcgggtcgt ggcgggagga 9000
   ctggggacag ctttcgggga cggccgtgcc gccccagggt gccgagcccc agagcaacgc 9060
   gggcccacga ccccatatcg gggacacgtt atttaccctg tttcgggccc ccgagttgct 9120
   ggcccccaac ggcgacctgt ataacgtgtt tgcctgggcc ttggacgtct tggccaaacg 9180
   cctccgttcc atgcacgtct ttatcctgga ttacgaccaa tcgcccgccg gctgccggga 9240
   cgccctgctg caacttacct ccgggatggt ccagacccac gtcaccaccc ccggctccat 9300
   accgacgata tgcgacctgg cgcgcacgtt tgcccgggag atgggggagg ctaactgagg 9360
   ggatcgatcc gtcctgtaag tctgcagaaa ttgatgatct attaaacaat aaagatgtcc 9420
   actaaaatgg aagtttttcc tgtcatactt tgttaagaag ggtgagaaca gagtacctac 9480
   attttgaatg gaaggattgg agctacgggg gtgggggtgg ggtgggatta gataaatgcc 9540
   tgctctttac tgaaggctct ttactattgc tttatgataa tgtttcatag ttggatatca 9600
   taatttaaac aagcaaaacc aaattaaggg ccagctcatt cctcccactc atgatctata 9660
   gatctataga tctctcgtgg gatcattgtt tttctcttga ttcccacttt gtggttctaa 9720
   gtactgtggt ttccaaatgt gtcagtttca tagcctgaag aacgagatca gcagcctctg 9780
   ttccacatac acttcattct cagtattgtt ttgccaagtt ctaattccat cagaagctga 9840
   ctctaggccg agctccaatt cgccctatag tgagtcgtat tacaattcac tggccgtcgt 9900
   tttacaacgt cgtgactggg aaaaccctgg cgttacccaa cttaatcgcc ttgcagcaca 9960
   tccccctttc gccagctggc gtaatagcga agaggcccgc accgatcgcc cttcccaaca 10020
   gttgcgcagc ctgaatggcg aatgggacgc gccctgtagc ggcgcattaa gcgcggcggg 10080
   tgtggtggtt acgcgcagcg tgaccgctac acttgccagc gccctagcgc ccgctccttt 10140
   cgctttcttc ccttcctttc tcgccacgtt cgccggcttt ccccgtcaag ctctaaatcg 10200
   ggggctccct ttagggttcc gatttagtgc tttacggcac ctcgacccca aaaaacttga 10260
   ttagggtgat ggttcacgta gtgggccatc gccctgatag acggtttttc gccctttgac 10320
   gttggagtcc acgttcttta atagtggact cttgttccaa actggaacaa cactcaaccc 10380
   tatctcggtc tattcttttg atttataagg gattttgccg atttcggcct attggttaaa 10440
   aaatgagctg atttaacaaa aatttaacgc gaattttaac aaaatattaa cgcttacaat 10500
   ttaggtggca cttttcgggg aaatgtgcgc ggaaccccta tttgtttatt tttctaaata 10560
   cattcaaata tgtatccgct catgagacaa taaccctgat aaatgcttca ataatattga 10620
   aaaaggaaga gtatgagtat tcaacatttc cgtgtcgccc ttattccctt ttttgcggca 10680
   ttttgccttc ctgtttttgc tcacccagaa acgctggtga aagtaaaaga tgctgaagat 10740
   cagttgggtg cacgagtggg ttacatcgaa ctggatctca acagcggtaa gatccttgag 10800
   agttttcgcc ccgaagaacg ttttccaatg atgagcactt ttaaagttct gctatgtggc 10860
   gcggtattat cccgtattga cgccgggcaa gagcaactcg gtcgccgcat acactattct 10920
   cagaatgact tggttgagta ctcaccagtc acagaaaagc atcttacgga tggcatgaca 10980
   gtaagagaat tatgcagtgc tgccataacc atgagtgata acactgcggc caacttactt 11040
   ctgacaacga tcggaggacc gaaggagcta accgcttttt tgcacaacat gggggatcat 11100
   gtaactcgcc ttgatcgttg ggaaccggag ctgaatgaag ccataccaaa cgacgagcgt 11160
   gacaccacga tgcctgtagc aatggcaaca acgttgcgca aactattaac tggcgaacta 11220
   cttactctag cttcccggca acaattaata gactggatgg aggcggataa agttgcagga 11280
   ccacttctgc gctcggccct tccggctggc tggtttattg ctgataaatc tggagccggt 11340
   gagcgtgggt ctcgcggtat cattgcagca ctggggccag atggtaagcc ctcccgtatc 11400
   gtagttatct acacgacggg gagtcaggca actatggatg aacgaaatag acagatcgct 11460
   gagataggtg cctcactgat taagcattgg taactgtcag accaagttta ctcatatata 11520
   ctttagattg atttaaaact tcatttttaa tttaaaagga tctaggtgaa gatccttttt 11580
   gataatctca tgaccaaaat cccttaacgt gagttttcgt tccactgagc gtcagacccc 11640
   gtagaaaaga tcaaaggatc ttcttgagat cctttttttc tgcgcgtaat ctgctgcttg 11700
   caaacaaaaa aaccaccgct accagcggtg gtttgtttgc cggatcaaga gctaccaact 11760
   ctttttccga aggtaactgg cttcagcaga gcgcagatac caaatactgt ccttctagtg 11820
   tagccgtagt taggccacca cttcaagaac tctgtagcac cgcctacata cctcgctctg 11880
   ctaatcctgt taccagtggc tgctgccagt ggcgataagt cgtgtcttac cgggttggac 11940
   tcaagacgat agttaccgga taaggcgcag cggtcgggct gaacgggggg ttcgtgcaca 12000
   cagcccagct tggagcgaac gacctacacc gaactgagat acctacagcg tgagctatga 12060
   gaaagcgcca cgcttcccga agggagaaag gcggacaggt atccggtaag cggcagggtc 12120
   ggaacaggag agcgcacgag ggagcttcca gggggaaacg cctggtatct ttatagtcct 12180
   gtcgggtttc gccacctctg acttgagcgt cgatttttgt gatgctcgtc aggggggcgg 12240
   agcctatgga aaaacgccag caacgcggcc tttttacggt tcctggcctt ttgctggcct 12300
   tttgctcaca tgttctttcc tgcgttatcc cctgattctg tggataaccg tattaccgcc 12360
   tttgagtgag ctgataccgc tcgccgcagc cgaacgaccg agcgcagcga gtcagtgagc 12420
   gaggaagcgg aagagcgccc aatacgcaaa ccgcctctcc ccgcgcgttg gccgattcat 12480
   taatgcagct ggcacgacag gtttcccgac tggaaagcgg gcagtgagcg caacgcaatt 12540
   aatgtgagtt agctcactca ttaggcaccc caggctttac actttatgct tccggctcgt 12600
   atgttgtgtg gaattgtgag cggataacaa tttcacacag gaaacagcta tgaccatgat 12660
   tacgccaagc gcgcaattaa ccctcactaa agggaacaaa agctgtcgag atctagatat 12720
   cgatggccat agagttacg 12739

<210> 8
<211> 15342
<212> DNA
<213> Artificial Sequence

<220>

<223> Description of Artificial Sequence: Rosa 26 locus
      targeted with targeting vector

<400> 8
aagcttctca cgtagcaacc agagctccag agccagcagc tgctgccgcc ttgtatactc 60
actcctgtga tccaacacag gagcaacctt ttctttaccc cacccccact tcttaacaca 120
cttttttttg gggggggggg gggaacaagt gctccatgct ggaaggattg gaactatgct 180
tttagaaagg aacaatccta aggtcacttt taaattgagg tctttgattt gaaaatcaac 240
aaataccaaa ttccaaatat tcgttttaat taaaccagca atgtggatat aagcattaag 300
ttttagtttt aaaaaggtca attttccaaa cattcagcaa tcatatttaa atttacagct 360
aggaacaaga gccttgggtc atgtcctacc aaagaacata actcaatatt ctacacatga 420
caatctgaat aaccttaaag cctctaatcc cataacaggc cacaaatttt ggacagagaa 480
ctaatgatcc tcctgagaaa actggaagaa atccagggaa aagaaattcc tgtgtcctcc 540
aaactcagaa atctctaatt atgtcagtat tctctgcttt agtcctaggt cagattgcac 600
acatctaaaa taacctctta aagttttcct cctagcgacc taaaccatta ttaatatcaa 660
attaaccatc aaaacacttt cctctcaata tgctgcacac aaacctcctc ctggaacctc 720
ctccatctgg atcctcccca atcaaaagta taggtattta acatataagc aaggaagtaa 780
tgtaaacatg accttggtca caaatatgtc atctaaaaac aatttagtca aggtatggag 840
gaaattcgag aacctgaatc tttttaagta ttttgagcac aggaacaatt ggcaaaagga 900
atccaggtat agacaaaacc cagagcccag agctctgggc gaaaaatgag ttgctggtga 960
agacgttaca caagtaacat gagaaagcag aaaatgcagg tcatccacgc acccctgacc 1020
caggccagca gggcgggctg cagcatcagt acacaggaga aagatcctta ttcctaagaa 1080
tgagaaaggc aaaggcgccc gatagaataa attagcatag aaggggcttt cccaggagtt 1140
aaaactttcc ttctgagcga ttacctacta aaaccagggc ttttgcccac taccatttac 1200
ctaggatctt ggcttgcacg gattcatagg ggcatatccc tccccctctt ctttagagtc 1260
gttcttaaaa gatcgctctc cacgccctag gcagggaaaa cgacaaaatc tggctcaatt 1320
ccaggctaga accctacaaa ttcaacaggg atatcgcaag gatactgggg catacgccac 1380
agggagtcca agaatgtgag gtgggggtgg cgaaggtaat gtctttggtg tgggaaaagc 1440
agcagccatc tgagatagga actggaaaac cagaggagag gcgttcagga agattatgga 1500
ggggaggact gggcccccac gagcgaccag agttgtcaca aggccgcaag aacaggggag 1560
gtggggggct cagggacaga aaaaaaagta tgtgtatttt gagagcaggg ttgggaggcc 1620
tctcctgaaa agggtataaa cgtggagtag gcaataccca ggcaaaaagg ggagaccaga 1680
gtagggggag gggaagagtc ctgacccagg gaagacatta aaaaggtagt ggggtcgact 1740
agatgaagga gagcctttct ctctgggcaa gagcggtgca atggtgtgta aaggtagctg 1800
agaagacgaa aagggcaagc atcttcctgc taccaggctg gggaggccca ggcccacgac 1860
cccgaggaga gggaacgcag ggagactgag gtgacccttc tttcccccgg ggcccggtcg 1920
tgtggttcgg tgtctctttt ctgttggacc cttaccttga cccaggcgct gccggggcct 1980
gggcccgggc tgcggcgcac ggcactcccg ggaggcagcg agactcgagt taggcccaac 2040
gcggcgccac ggcgtttcct ggccgggaat ggcccgtacc cgtgaggtgg gggtgggggg 2100
cagaaaaggc ggagcgagcc cgagcgggga gggggagggc caggggcgga gggggccggc 2160
actactgtgt tggcggactg gcgggactag ggctgcgtga gtctctgagc gcaggcgggc 2220
ggcggccgcc cctcccccgg cggcggcagc ggcggcagcg gcggcagctc actcagcccg 2280
ctgcccgagc ggaaacgcca ctgaccgcac ggggattccc agtgccggcg ccaggggcac 2340
gcgggacacg ccccctcccg ccgcgccatt ggcctctccg cccaccgccc cacacttatt 2400
ggccggtgcg ccgccaatca gcggaggctg ccggggccgc ctaaagaaga ggctgtgctt 2460
tggggctccg gctcctcaga gagcctcggc taggtagggg atcgggactc tggcgggagg 2520
gcggcttggt gcgtttgcgg ggatgggcgg ccgcggcagg ccctccgagc gtggtggagc 2580
cgttctgtga gacagccggg tacgagtcgt gacgctggaa ggggcaagcg ggtggtgggc 2640
aggaatgcgg tccgccctgc agcaaccgga gggggaggga gaagggagcg gaaaagtctc 2700
caccggacgc ggccatggct cggggggggg ggggcagcgg aggagcgctt ccggccgacg 2760
tctcgtcgct gattggcttc ttttcctccc gccgtgtgtg aaaacacaaa tggcgtgttt 2820
tggttggcgt aaggcgcctg tcagttaacg gcagccggag tgcgcagccg ccggcagcct 2880
cgctctgccc actgggtggg gcgggaggta ggtggggtga ggcgagctgg acgtgcgggc 2940
gcggtcggcc tctggcgggg cgggggaggg gagggagggt cagcgaaagt agctcgcgcg 3000
cgagcggccg cccaccctcc ccttcctctg ggggagtcgt tttacccgcc gccggccggg 3060
cctcgtcgtc tgattggctc tcggggccca gaaaactggc ccttgccatt ggctcgtgtt 3120
cgtgcaagtt gagtccatcc gccggccagc gggggcggcg aggaggcgct cccaggttcc 3180
ggccctcccc tcggccccgc gccgcagagt ctggccgcgc gcccctgcgc aacgtggcag 3240
gaagcgcgcg ctgggggcgg ggacgggcag tagggctgag cggctgcggg gcgggtgcaa 3300
gcacgtttcc gacttgagtt gcctcaagag gggcgtgctg agccagacct ccatcgcgca 3360
ctccggggag tggagggaag gagcgagggc tcagttgggc tgttttggag gcaggaagca 3420

cttgctctcc caaagtcgct ctgagttgtt atcagtaagg gagctgcagt ggagtaggcg 3480
gggagaaggc cgcacccttc tccggagggg ggaggggagt gttgcaatac ctttctggga 3540
gttctctgct gcctcctggc ttctgaggac cgccctgggc ctgggagaat cccttccccc 3600
tcttccctcg tgatctgcaa ctccagtctt tctaggtaac cgatatccct gcaggggtga 3660
cctgcacgtc tagggcgcag tagtccaggg tttccttgat gatgtcatac ttatcctgtc 3720
cctttttttt ccacagctcg cggttgagga caaactcttc gcggtctttc cagtactcct 3780
gcaggtgact gactgagtcg actaggggta accataactt cgtataatgt atgctatacg 3840
aagttattta taatctagaa ctagtggatc cacgattcga gggcccctgc aggtcaattc 3900
taccgggtag gggaggcgct tttcccaagg cagtctggag catgcgcttt agcagccccg 3960
ctgggcactt ggcgctacac aagtggcctc tggcctcgca cacattccac atccaccggt 4020
aggcgccaac cggctccgtt ctttggtggc cccttcgcgc caccttctac tcctccccta 4080
gtcaggaagt tcccccccgc cccgcagctc gcgtcgtgca ggacgtgaca aatggaagta 4140
gcacgtctca ctagtctcgt gcagatggac agcaccgctg agcaatggaa gcgggtaggc 4200
ctttggggca gcggccaata gcagctttgc tccttcgctt tctgggctca gaggctggga 4260
aggggtgggt ccgggggcgg gctcagggcg ggctcagggg cggggcgggc gcccgaaggt 4320
cctccggagg cccggcattc tgcacgcttc aaaagcgcac gtctgccgcg ctgttctcct 4380
cttcctcatc tccgggcctt tcgacctgca gccaatatgg gatcggccat tgaacaagat 4440
ggattgcacg caggttctcc ggccgcttgg gtggagaggc tattcggcta tgactgggca 4500
caacagacaa tcggctgctc tgatgccgcc gtgttccggc tgtcagcgca ggggcgcccg 4560
gttctttttg tcaagaccga cctgtccggt gccctgaatg aactgcagga cgaggcagcg 4620
cggctatcgt ggctggccac gacgggcgtt ccttgcgcag ctgtgctcga cgttgtcact 4680
gaagcgggaa gggactggct gctattgggc gaagtgccgg ggcaggatct cctgtcatct 4740
caccttgctc ctgccgagaa agtatccatc atggctgatg caatgcggcg gctgcatacg 4800
cttgatccgg ctacctgccc attcgaccac caagcgaaac atcgcatcga gcgagcacgt 4860
actcggatgg aagccggtct tgtcgatcag gatgatctgg acgaagagca tcaggggctc 4920
gcgccagccg aactgttcgc caggctcaag gcgcgcatgc ccgacggcga ggatctcgtc 4980
gtgacccatg gcgatgcctg cttgccgaat atcatggtgg aaaatggccg cttttctgga 5040
ttcatcgact gtggccggct gggtgtggcg gaccgctatc aggacatagc gttggctacc 5100
cgtgatattg ctgaagagct tggcggcgaa tgggctgacc gcttcctcgt gctttacggt 5160
atcgccgctc ccgattcgca gcgcatcgcc ttctatcgcc ttcttgacga gttcttctga 5220
ggggatcgat ccgctgtaag tctgcagaaa ttgatgatct attaaacaat aaagatgtcc 5280
actaaaatgg aagtttttcc tgtcatactt tgttaagaag ggtgagaaca gagtacctac 5340
attttgaatg gaaggattgg agctacgggg gtgggggtgg ggtgggatta gataaatgcc 5400
tgctctttac tgaaggctct ttactattgc tttatgataa tgtttcatag ttggatatca 5460
taatttaaac aagcaaaacc aaattaaggg ccagctcatt cctcccactc atgatctata 5520
gatctataga tctctcgtgg gatcattgtt tttctcttga ttcccacttt gtggttctaa 5580
gtactgtggt ttccaaatgt gtcagtttca tagcctgaag aacgagatca gcagcctctg 5640
ttccacatac acttcattct cagtattgtt ttgccaagtt ctaattccat cagaagctga 5700
ctctagatcc tgcaggaatt catatgataa cttcgtataa tgtatgctat acgaagttat 5760
ggcgcgccgg taaccgaagt tcctatactt tctagagaat aggaacttcg gaataggaac 5820
ttcaagctta agcgctagaa gatgggcggg agtcttctgg gcaggcttaa aggctaacct 5880
ggtgtgtggg cgttgtcctg caggggaatt gaacaggtgt aaaattggag ggacaagact 5940
tcccacagat tttcggtttt gtcgggaagt tttttaatag gggcaaataa ggaaaatggg 6000
aggataggta gtcatctggg gttttatgca gcaaaactac aggttattat tgcttgtgat 6060
ccgcctcgga gtattttcca tcgaggtaga ttaaagacat gctcacccga gttttatact 6120
ctcctgcttg agatccttac tacagtatga aattacagtg tcgcgagtta gactatgtaa 6180
gcagaatttt aatcattttt aaagagccca gtacttcata tccatttctc ccgctccttc 6240
tgcagcctta tcaaaaggta ttttagaaca ctcattttag ccccatttc atttattata 6300
ctggcttatc caacccctag acagagcatt ggcattttcc ctttcctgat cttagaagtc 6360
tgatgactca tgaaaccaga cagattagtt acatacacca caaatcgagg ctgtagctgg 6420
ggcctcaaca ctgcagttct tttataactc cttagtacac tttttgttga tcctttgcct 6480
tgatccttaa ttttcagtgt ctatcacctc tcccgtcagt ggtgttccac atttgggcct 6540
attctcagtc cagggagttt tacaacaata gatgtattga gaatccaacc taaagcttaa 6600
ctttccactc ccatgaatgc ctctctcctt tttctccatt tataaactga gctattaacc 6660
attaatggtt ccaggtggat gtctcctccc catattacct gatgtatctt acatattgcc 6720
aggctgatat tttaagacat taaaaggtat atttcattat tgagccacat ggtattgatt 6780
actgcttact aaaattttgt cattgtacac atctgtaaaa ggtggttcct tttggaatgc 6840
aaagttcagg tgtttgttgt ctttcctgac ctaaggtctt gtgagcttgt attttttcta 6900
tttaagcagt gctttctctt ggactggctt gactcatggc attctacacg ttattgctgg 6960
tctaaatgtg attttgccaa gcttcttcag gacctataat tttgcttgac ttgtagccaa 7020
acacaagtaa aatgattaag caacaaatgt atttgtgaag cttggttttt aggttgttgt 7080

gttgtgtgtg cttgtgctct ataataatac tatccagggg ctggagaggt ggctcggagt 7140
tcaagagcac agactgctct tccagaagtc ctgagttcaa ttcccagcaa ccacatggtg 7200
gctcacaacc atctgtaatg ggatctgatg ccctcttctg gtgtgtctga agaccacaag 7260
tgtattcaca ttaaataaat aaatcctcct tcttcttctt tttttttttt ttaaagagaa 7320
tactgtctcc agtagaattt actgaagtaa tgaaatactt tgtgtttgtt ccaatatggt 7380
agccaataat caaattactc tttaagcact ggaaatgtta ccaaggaact aatttttatt 7440
tgaagtgtaa ctgtggacag aggagccata actgcagact tgtgggatac agaagaccaa 7500
tgcagacttt aatgtctttt ctcttacact aagcaataaa gaaataaaaa ttgaacttct 7560
agtatcctat ttgtttaaac tgctagcttt acttaacttt tgtgcttcat ctatacaaag 7620
ctgaaagcta agtctgcagc cattactaaa catgaaagca agtaatgata attttggatt 7680
tcaaaaatgt agggccagag tttagccagc cagtggtggt gcttgccttt atgcctttaa 7740
tcccagcact ctggaggcag agacaggcag atctctgagt ttgagcccag cctggtctac 7800
acatcaagtt ctatctagga tagccaggaa tacacacaga aaccctgttg gggagggggg 7860
ctctgagatt tcataaaatt ataattgaag cattccctaa tgagccacta tggatgtggc 7920
taaatccgtc tacctttctg atgagatttg ggtattattt tttctgtctc tgctgttggt 7980
tgggtctttt gacactgtgg gctttcttta aagcctcctt cctgccatgt ggtctcttgt 8040
ttgctactaa cttcccatgg cttaaatggc atggcttttt gccttctaag ggcagctgct 8100
gagatttgca gcctgatttc cagggtgggg ttgggaaatc tttcaaacac taaaattgtc 8160
ctttaatttt ttttttaaaa aatgggttat ataataaacc tcataaaata gttatgagga 8220
gtgaggtgga ctaatattaa atgagtccct cccctataaa agagctatta aggctttttg 8280
tcttatactt aacttttttt ttaaatgtgg tatctttaga accaagggtc ttagagtttt 8340
agtatacaga aactgttgca tcgcttaatc agattttcta gtttcaaatc cagagaatcc 8400
aaattcttca cagccaaagt caaattaaga atttctgact tttaatgtta atttgcttac 8460
tgtgaatata aaaatgatag cttttcctga ggcagggtct cactatgtat ctctgcctga 8520
tctgcaacaa gatatgtaga ctaaagttct gcctgctttt gtctcctgaa tactaaggtt 8580
aaaatgtagt aatacttttg gaacttgcag gtcagattct tttatagggg acacactaag 8640
ggagcttggg tgatagttgg taaaatgtgt ttcaagtgat gaaaacttga attattatca 8700
ccgcaaccta ctttttaaaa aaaaaagcca ggcctgttag agcatgctta agggatccct 8760
aggacttgct gagcacacaa gagtagttac ttggcaggct cctggtgaga gcatatttca 8820
aaaaacaagg cagacaacca agaaactaca gttaaggtta cctgtcttta aaccatctgc 8880
atatacacag ggatattaaa atattccaaa taatatttca ttcaagtttt cccccatcaa 8940
attgggacat ggatttctcc ggtgaatagg cagagttgga aactaaacaa atgttggttt 9000
tgtgatttgt gaaattgttt tcaagtgata gttaaagccc atgagataca gaacaaagct 9060
gctatttcga ggtctcttgg tttatactca gaagcacttc tttgggtttc cctgcactat 9120
cctgatcatg tgctaggcct accttaggct gattgttgtt caaataaact taagtttcct 9180
gtcaggtgat gtcatatgat ttcatatatc aaggcaaaac atgttatata tgttaaacat 9240
ttgtacttaa tgtgaaagtt aggtctttgt gggtttgatt tttaattttc aaaacctgag 9300
ctaaataagt catttttaca tgtcttacat ttggtggaat tgtataattg tggtttgcag 9360
gcaagactct ctgacctagt aaccctacct atagagcact ttgctgggtc acaagtctag 9420
gagtcaagca tttcaccttg aagttgagac gttttgttag tgtatactag tttatatgtt 9480
ggaggacatg tttatccaga agatattcag gactattttt gactgggcta aggaattgat 9540
tctgattagc actgttagtg agcattgagt ggcctttagg cttgaattgg agtcacttgt 9600
atatctcaaa taatgctggc ctttttttaaa aagcccttgt tctttatcac cctgtttctt 9660
acataatttt tgttcaaaga aatacttgtt tggatctcct tttgacaaca atagcatgtt 9720
ttcaagccat attttttttc cttttttttt ttttttttgg tttttcgaga cagggtttct 9780
ctgtatagcc ctggctgtcc tggaactcac tttgtagacc aggctggcct cgaactcaga 9840
aatccgcctg cctctgcctc ctgagtgccg ggattaaagg cgtgcaccac cacgcctggc 9900
taagttggat attttgttat ataactataa ccaatactaa ctccactggg tggattttta 9960
attcagtcag tagtcttaag tggtctttat tggcccttca ttaaaatcta ctgttcactc 10020
taacagaggc tgttggtact agtggcactt aagcaacttc ctacggatat actagcagat 10080
taagggtcag ggatagaaac tagtctagcg ttttgtatac ctaccagctt tatactacct 10140
tgttctgata gaaatatttc aggacatcta gagtgtacta taaggttgat ggtaagctta 10200
taaggaactt gaaagtggag taactactcc atttctctga ggggagaatt aaaatttttg 10260
accaagtgtt gttgagccac tgagaatggt ctcagaacat aacttcttaa ggaaccttcc 10320
cagattgccc tcaacactgc accacatttg gtcctgcttg aacattgcca tggctcttaa 10380
agtcttaatt aagaatatta attgtgtaat tattgttttt cctcctttag atcattcctt 10440
gaggacagga cagtgcttgt ttaaggctat atttctgctg tctgagcagc aacaggtctt 10500
cgagatcaac atgatgttca taatcccaag atgttgccat ttatgttctc agaagcaagc 10560
agaggcatga tggtcagtga cagtaatgtc actgtgttaa atgttgctat gcagtttgga 10620
tttttctaat gtagtgtagg tagaacatat gtgttctgta tgaattaaac tcttaagtta 10680
caccttgtat aatccatgca atgtgttatg caattaccat tttaagtatt gtagctttct 10740

ttgtatgtga ggataaaggt gtttgtcata aaatgttttg aacatttccc caaagttcca 10800
aattataaaa ccacaacgtt agaacttatt tatgaacaat ggttgtagtt tcatgctttt 10860
aaaatgctta attattcaat taacaccgtt tgtgttataa tatatataaa actgacatgt 10920
agaagtgttt gtccagaaca tttcttaaat gtatactgtc tttagagagt ttaatatagc 10980
atgtcttttg caacatacta acttttgtgt tggtgcgagc aatattgtgt agtcattttg 11040
aaaggagtca tttcaatgag tgtcagattg ttttgaatgt tattgaacat tttaaatgca 11100
gacttgttcg tgttttagaa agcaaaactg tcagaagctt tgaactagaa attaaaaagc 11160
tgaagtattt cagaagggaa ataagctact tgctgtatta gttgaaggaa agtgtaatag 11220
cttagaaaat ttaaaaccat atagttgtca ttgctgaata tctggcagat gaaaagaaat 11280
actcagtggt tcttttgagc aatataacag cttgttatat taaaaatttt ccccacagat 11340
ataaactcta atctataact cataaatgtt acaaatggat gaagcttaca aatgtggctt 11400
gacttgtcac tgtgcttgtt ttagttatgt gaaagtttgg caataaacct atgtcctaaa 11460
tagtcaaact gtggaatgac tttttaatct attggtttgt ctagaacagt tatgttgcca 11520
tttgccctaa tggtgaaaga aaaagtgggg agtgccttgg cactgttcat ttgtggtgtg 11580
aaccaaagag gggggcatgc acttacactt caaacatcct tttgaaagac tgacaagttt 11640
gggtcttcac agttggaatt gggcatccct tttgtcaggg agggagggag ggagggaggc 11700
tggcttgtta tgctgacaag tgtgattaaa ttcaaacttt gaggtaagtt ggaggaactt 11760
gtacattgtt aggagtgtga caatttggac tcttaatgat ttggtcatac aaaatgaacc 11820
tagaccaact tctggaagat gtatataata actccatgtt acattgattt cacctgacta 11880
atacttatcc cttatcaatt aaatacagaa gatgccagcc atctgggcct tttaacccag 11940
aaatttagtt tcaaactcct aggttagtgt tctcactgag ctacatcctg atctagtcct 12000
gaaaatagga ccaccatcac cccaaaaaaa atctcaaata agatttatgc tagtgtttca 12060
aaattttagg aataggtaag attagaaagt tttaaatttt gagaaatggc ttctctagaa 12120
agatgtacat agtgaacact gaatggctcc taaagagcct agaaaactgg tactgagcac 12180
acaggactga gaggtctttc ttgaaaagca tgtattgctt tacgtgggtc acagaaggca 12240
ggcaggaaga acttgggctg aaactggtgt cttaagtggc taacatcttc acaactgatg 12300
agcaagaact ttatcctgat gcaaaaacca tccaaacaaa ctaagtgaaa ggtggcaatg 12360
gatcccaggc tgctctagag gaggacttga cttctcatcc catcacccac accagatagc 12420
tcatagactg ccaattaaca ccagcttcta gcctccacag gcacctgcac tggtacacat 12480
aatttcacac aaacacagta agaagccttc cacctggcat ggtattgctt atctttagtt 12540
cccaacactt gggaggcaga ggccagccag ggctatgtga caaaaacctt gtctagagga 12600
gaaacttcat agcttatttc ctattcacgt aaccaggtta gcaaaattta ccagccagag 12660
atgaagctaa cagtgtccac tatatttgta gtgttttaag tcaatttttt aaatatactt 12720
aatagaatta aagctatggt gaaccaagta caaacctggt gtattaactt gagaacttag 12780
cataaaaagt agttcatttg ttcagtaaat attaaatgct tactggcaaa gattatgtca 12840
ggaacttggt aaatggtgat gaaacaatca tagttgtaca tcttggttct gtgatcacct 12900
tggtttgagg taaaagtggt tcctttgatc aaggatggaa ttttaagttt atattcaatc 12960
aataatgtat tattttgtga ttgcaaaatt gcctatctag ggtataaaac ctttaaaaat 13020
ttcataatac cagttcattc tccagttact aattccaaaa agccactgac tatggtgcca 13080
atgtggattc tgttctcaaa ggaaggattg tctgtgccct ttattctaat agaaacatca 13140
cactgaaaat ctaagctgaa agaagccaga ctttcctaaa taaataactt tccataaagc 13200
tcaaacaagg attactttta ggaggcactg ttaaggaact gataagtaat gaggttactt 13260
atataatgat agtcccacaa gactatctga ggaaaaatca gtacaactcg aaaacagaac 13320
aaccagctag gcaggaataa cagggctccc aagtcaggag gtctatccaa cacccttttc 13380
tgttgagggc cccagaccta catattgtat acaaacaggg aggtgggtga ttttaactct 13440
cctgaggtac cttggtaaat ctttgtcctg agtaagcagt acagtgtaca gtttacattt 13500
tcatttaaag atacattagc tccctctacc ccctaagact gacaggcact ttgggggtgg 13560
ggagggcttt ggaaaataac gcttccatac actaaaagag aaatttcttt aattaggctt 13620
gttggttcca tacatctact ggtgtttcta ctacttagta atattataat agtcacacaa 13680
gcatctttgc tctgtttagg ttgtatattt attttaaggc agatgataaa actgtagatc 13740
ttaagggatg cttctgcttc tgagatgata caaagaattt agaccataaa acagtaggtt 13800
gcacaagcaa tagaatatgg cctaaagtgt tctgacactt agaagccaag cagtgtaggc 13860
ttcttaagaa ataccattac aatcaccttg ctagaaatca agcattctgg agtggtcaag 13920
cagtgtaacc tgtactgtaa gttacttttc tgctattttt ctcccaaagc aagttcttta 13980
tgctgatatt tccagtgtta ggaactacaa atattaataa gttgtcttca ctcttttctt 14040
taccaaggag ggtctcttcc ttcatcttga tctgaaggat gaacaaaggc ttgagcagtg 14100
cgctttagaa gataaactgc agcatgaagg cccccgatgt tcacccagac tacatggacc 14160
tttcgccaca catgtcccat tccagataag gcctggcaca cacaaaaaac ataagtcatt 14220
aggctaccag tctgattcta aaacaaccta aaatcttccc acttaaatgc tatgggtggt 14280
gggttggaaa gttgactcag aaaatcactt gctgttttta gagaggatct gggttcagtt 14340
tctgatacat tgtggcttac aactataact ccagttctag gggtccatcc caacatcctc 14400

ttctgttgag ggcaccaaat aaatgtattg tgtacaaaca gggaggtgag tgatttaact 14460
ctcgtgtata gtaccttggt aaaacatttc ttgtcctgag taagcagtac agctctgcct 14520
gtccctggtc tacagacacg gctcatttcc cgaaggcaag ctggatagag attccaattt 14580
ctcttcttgg atcccatcct ataaaagaag gtcaagttta atctattgca aaaggtaaat 14640
aggtagtttc ttacatgaga caagaacaaa tcttaggtgt gaagcagtca tcttttacag 14700
gccagagcct ctattctatg ccaatgaagg aaactgttag tccagtgtta tagagttagt 14760
ccagtgtata gttttctatc agaacacttt ttttttaaac aactgcaact tagcttattg 14820
aagacaaacc acgagtagaa atctgtccaa gaagcaagtg cttctcagcc tacaatgtgg 14880
aataggacca tgtaatggta cagtgagtga aatgaattat ggcatgtttt tctgactgag 14940
aagacagtac aataaaaggt aaactcatgg tatttattta aaaagaatcc aatttctacc 15000
tttttccaaa tggcatatct gttacaataa tatccacaga agcagttctc agtgggaggt 15060
tgcagatatc ccactgaaca gcatcaatgg gcaaacccca ggttgttttt ctgtggagac 15120
aaaggtaaga tatttcaata tattttccca agctaatgag atggctcagc aaataatggt 15180
actggccatt aagtctcatg acctgagctt gatcctcagg gaccatgtgg tacaaggaga 15240
gacctaaatc cttcagttgg acttcaatct tctaccctca tgtccacaca caaataaata 15300
caataaaaaa cattctgcag tcgaatttct aaaagggcga at 15342

<210> 9
<211> 1823
<212> DNA
<213> Artificial Sequence

<220>
<223> Description of Artificial Sequence: PGK-neo-pA

<400> 9
ggtcaattct accgggtagg ggaggcgctt ttcccaaggc agtctggagc atgcgcttta 60
gcagccccgc tgggcacttg gcgctacaca agtggcctct ggcctcgcac acattccaca 120
tccaccggta ggcgccaacc ggctccgttc tttggtggcc ccttcgcgcc accttctact 180
cctcccctag tcaggaagtt cccccccgcc ccgcagctcg cgtcgtgcag gacgtgacaa 240
atggaagtag cacgtctcac tagtctcgtg cagatggaca gcaccgctga gcaatggaag 300
cgggtaggcc tttggggcag cggccaatag cagctttgct ccttcgcttt ctgggctcag 360
aggctgggaa ggggtgggtc cgggggcggg ctcagggcgg gctcaggggc ggggcgggcg 420
cccgaaggtc ctccggaggc ccggcattct gcacgcttca aaagcgcacg tctgccgcgc 480
tgttctcctc ttcctcatct ccgggccttt cgacctgcag ccaatatggg atcggccatt 540
gaacaagatg gattgcacgc aggttctccg gccgcttggg tggagaggct attcggctat 600
gactgggcac aacagacaat cggctgctct gatgccgccg tgttccggct gtcagcgcag 660
gggcgcccgg ttctttttgt caagaccgac ctgtccggtg ccctgaatga actgcaggac 720
gaggcagcgc ggctatcgtg gctggccacg acgggcgttc cttgcgcagc tgtgctcgac 780
gttgtcactg aagcgggaag ggactggctg ctattgggcg aagtgccggg gcaggatctc 840
ctgtcatctc accttgctcc tgccgagaaa gtatccatca tggctgatgc aatgcggcgg 900
ctgcatacgc ttgatccggc tacctgccca ttcgaccacc aagcgaaaca tcgcatcgag 960
cgagcacgta ctcggatgga agccggtctt gtcgatcagg atgatctgga cgaagagcat 1020
caggggctcg cgccagccga actgttcgcc aggctcaagg cgcgcatgcc cgacggcgag 1080
gatctcgtcg tgacccatgg cgatgcctgc ttgccgaata tcatggtgga aaatggccgc 1140
ttttctggat tcatcgactg tggccggctg ggtgtggcgg accgctatca ggacatagcg 1200
ttggctaccc gtgatattgc tgaagagctt ggcggcgaat gggctgaccg cttcctcgtg 1260
ctttacggta tcgccgctcc cgattcgcag cgcatcgcct tctatcgcct tcttgacgag 1320
ttcttctgag gggatcgatc cgctgtaagt ctgcagaaat tgatgatcta ttaaacaata 1380
aagatgtcca ctaaaatgga agtttttcct gtcatacttt gttaagaagg gtgagaacag 1440
agtacctaca ttttgaatgg aaggattgga gctacggggg tgggggtggg gtgggattag 1500
ataaatgcct gctctttact gaaggctctt tactattgct ttatgataat gtttcatagt 1560
tggatatcat aatttaaaca agcaaaacca aattaagggc cagctcattc ctcccactca 1620
tgatctatag atctatagat ctctcgtggg atcattgttt ttctcttgat tcccactttg 1680
tggttctaag tactgtggtt tccaaatgtg tcagtttcat agcctgaaga acgagatcag 1740
cagcctctgt tccacataca cttcattctc agtattgttt tgccaagttc taattccatc 1800
agaagctgac tctagatcct gca 1823

<210> 10

<211> 1867
<212> DNA
<213> Artificial Sequence

<220>
<223> Description of Artificial Sequence: CAGGS-Intron

<400> 10
tcgacattga ttattgacta gttattaata gtaatcaatt acggggtcat tagttcatag 60
cccatatatg gagttccgcg ttacataact tacggtaaat ggcccgcctg gctgaccgcc 120
caacgacccc cgcccattga cgtcaataat gacgtatgtt cccatagtaa cgccaatagg 180
gactttccat tgacgtcaat gggtggacta tttacggtaa actgcccact tggcagtaca 240
tcaagtgtat catatgccaa gtacgccccc tattgacgtc aatgacggta aatggcccgc 300
ctggcattat gcccagtaca tgaccttatg ggactttcct acttggcagt acatctacgt 360
attagtcatc gctattacca tgggtcgagg tgagccccac gttctgcttc actctcccca 420
tctccccccc ctccccaccc ccaattttgt atttatttat tttttaatta ttttgtgcag 480
cgatgggggc gggggggggg ggggcgcgcg ccaggcgggg cggggcgggg cgaggggcgg 540
ggcggggcga ggcggagagg tgcggcggca gccaatcaga gcggcgcgct ccgaaagttt 600
ccttttatgg cgaggcggcg gcggcggcgg ccctataaaa agcgaagcgc gcggcgggcg 660
ggagtcgctg cgttgccttc gccccgtgcc ccgctccgcg ccgcctcgcg ccgcccgccc 720
cggctctgac tgaccgcgtt actcccacag gtgagcgggc gggacggccc ttctcctccg 780
ggctgtaatt agcgcttggt ttaatgacgg ctcgtttctt ttctgtggct gcgtgaaagc 840
cttaaagggc tccgggaggg ccctttgtgc ggggggagc ggctcggggg gtgcgtgcgt 900
gtgtgtgtgc gtggggagcg ccgcgtgcgg cccgcgctgc ccggcggctg tgagcgctgc 960
gggcgcggcg cggggctttg tgcgctccgc gtgtgcgcga ggggagcgcg gccgggggcg 1020
gtgccccgcg gtgcgggggg gctgcgaggg gaacaaaggc tgcgtgcggg gtgtgtgcgt 1080
gggggggtga gcagggggtg tgggcgcggc ggtcggctg taaccccccc ctgcaccccc 1140
ctccccgagt tgctgagcac ggcccggctt cgggtgcggg gctccgtgcg gggcgtggcg 1200
cggggctcgc cgtgccgggc ggggggtggc ggcaggtggg ggtgccgggc ggggcggggc 1260
cgcctcgggc cggggagggc tcgggggagg ggcgcggcgg ccccggagcg ccggcggctg 1320
tcgaggcgcg gcgagccgca gccattgcct tttatggtaa tcgtgcgaga gggcgcaggg 1380
acttcctttg tcccaaatct ggcggagccg aaatctggga ggcgccgccg caccccctct 1440
agcgggcgcg ggcgaagcgg tgcggcgccg gcaggaagga aatgggcggg gagggccttc 1500
gtgcgtcgcc gcgccgccgt ccccttctcc atctccagcc tcggggctgc cgcaggggga 1560
cggctgcctt cgggggggac ggggcagggc ggggttcggc ttctggcgtg tgaccggcgg 1620
ctctagtaag cgttggggtg agtactccct ctcaaaagcg ggcatgactt ctgcgctaag 1680
attgtcagtt tccaaaaacg aggaggattt gatattcacc tggcccgcgg tgatgccttt 1740
gagggtggcc gcgtccatct ggtcagaaaa gacaatcttt ttgttgtcaa gcttgaggtg 1800
tggcaggctt gagatctggc catacacttg agtgacattg acatccactt tgcctttctc 1860
tccacag 1867

<210> 11
<211> 703
<212> DNA
<213> Artificial Sequence

<220>
<223> Description of Artificial Sequence: ZsGreen ORF

<400> 11
cgccaccatg gcccagtcca agcacggcct gaccaaggag atgaccatga agtaccgcat 60
ggagggctgc gtggacggcc acaagttcgt gatcaccggc gagggcatcg gctacccctt 120
caagggcaag caggccatca acctgtgcgt ggtggagggc ggccccttgc ccttcgccga 180
ggacatcttg tccgccgcct tcatgtacgg caaccgcgtg ttcaccgagt acccccagga 240
catcgtcgac tacttcaaga actcctgccc cgccggctac acctgggacc gctccttcct 300
gttcgaggac ggcgccgtgt gcatctgcaa cgccgacatc accgtgagcg tggaggagaa 360
ctgcatgtac cacgagtcca agttctacgg cgtgaacttc cccgccgacg gccccgtgat 420
gaagaagatg accgacaact gggagccctc ctgcgagaag atcatccccg tgcccaagca 480
gggcatcttg aagggcgacg tgagcatgta cctgctgctg aaggacggtg gccgcttgcg 540
ctgccagttc gacaccgtgt acaaggccaa gtccgtgccc cgcaagatgc ccgactggca 600

cttcatccag cacaagctga cccgcgagga ccgcagcgac gccaagaacc agaagtggca 660
cctgaccgag cacgccatcg cctccggctc cgccttgccc tga 703

<210> 12
<211> 36
<212> DNA
<213> Artificial Sequence

<220>
<223> Description of Artificial Sequence: pA

<400> 12
aaataaagca atagcatcac aaatttcaca aataaa 36